# EUROPEAN PATENT SPECIFICATION

(11) **EP 4 106 747 B1**
(45) Date of publication and mention of the grant of the patent: **14.10.2026**
(21) Application number: 21757077.9
(22) Date of filing: 19.02.2021
(51) Int. Cl.: A61K 31/37, A61K 36/61, A61K 9/48, A61P 35/00, A61P 37/02, C07D 493/22, A61K 39/395, C07K 16/28

(54) **CASTALAGIN FOR USE IN ENHANCING THE ANTI-TUMOUR IMMUNE RESPONSE IN SUBJECTS SUFFERING FROM CANCER**
CASTALAGIN ZUR VERWENDUNG BEI DER VERSTÄRKUNG DER ANTITUMOR-IMMUNANTWORT BEI PATIENTEN, DIE AN KREBS LEIDEN
CASTALAGINE À UTILISER POUR RENFORCER LA RÉPONSE IMMUNITAIRE ANTITUMORALE CHEZ LES SUJETS ATTEINTS D'UN CANCER

(30) Priority: 20.02.2020 US 202062979327 P
(43) Date of publication of application: 28.12.2022
(73) Proprietor: The Royal Institution for the Advancement of Learning / McGill University, Montréal, Québec H3A 0G4 (CA); Val-Chum, Limited Partnership, Québec QC G1P 4P5 (CA); Université Laval, Québec, Québec G1V 0A6 (CA)
(72) Inventor: ROUTY, Bertrand, Montréal, Québec H3W 2L6 (CA); MARETTE, André, Québec, Québec G1V 4G5 (CA); MESSAOUDENE, Meriem, Westmount, Québec H3Z 2B1 (CA); CASTAGNER, Bastien, Montréal, Québec H2W 2J3 (CA); PIDGEON, Reilly, Montréal, Québec H3C 0P3 (CA)
(74) Representative: Cabinet Becker et Associés
(86) International application number: PCT/CA2021/050183
(87) International publication number: WO 2021/163802

(56) References cited:
- WO-A1-2014/043708
- WO-A1-2018/213732
- WO-A1-2019/178542
- BERTRAND ROUTY; EMMANUELLE LE CHATELIER; LISA DEROSA; CONNIE P M DUONG; MARYAM TIDJANI ALOU; ROMAIN DAILLÈRE; AURÉLIE FLUCKIGER; M: "Gut microbiome influences efficacy of PD-1-based immunotherapy against epithelial tumors", SCIENCE, vol. 359, no. 6371, 5 January 2018 (2018-01-05), US, pages 91 - 97, XP055554928, ISSN: 0036-8075, DOI: 10.1126/science.aan3706
- AUZANNEAU CÉLINE ET AL: "The Polyphenolic Ellagitannin Vescalagin Acts As a Preferential Catalytic Inhibitor of the [alpha] Isoform of Human DNA Topoisomerase II", MOLECULAR PHARMACOLOGY, 1 July 2012 (2012-07-01), XP093115533, Retrieved from the Internet <URL:https://doi.org/10.1124/mol.111.077537> [retrieved on 20240103]
- PERCHELLET J P ET AL: "Antitumor-Promoting Effects of Gallotannins, Ellagitannins, and Flavonoids in Mouse Skin In Vivo", CHEMISTRY, PROCESS DESIGN, AND SAFETY FOR THE NITRATION INDUSTRY /ACS /SYMPOSIUM SERIES, AMERICAN CHEMICAL SOCIETY/OXFORD UNIVERSITY PRESS, US, vol. 546, 1 January 1994 (1994-01-01), pages 303 - 327, XP008109699, ISSN: 0097-6156
- DANIELA FRACASSETTI ET AL: "Ellagic acid derivatives, ellagitannins, proanthocyanidins and other phenolics, vitamin C and antioxidant capacity of two powder products from camu-camu fruit (Myrciaria dubia)", FOOD CHEMISTRY, vol. 139, no. 1-4, 1 August 2013 (2013-08-01), pages 578 - 588, XP055116812, ISSN: 0308-8146, DOI: 10.1016/j.foodchem.2013.01.121
- KUO, PO-LINA; HSU, YA-LINGB; LIN, TA-CHENC; CHANG, JIUNN-KAEA; LIN, CHUN-CHING: "Induction of cell cycle arrest and apoptosis in human non-small cell lung cancer A549 cells by casuarinin from the bark of Terminalia arjuna Linn", ANTI-CANCER DRUGS, vol. 16, no. 4, 31 March 2005 (2005-03-31), US, pages 409 - 415, XP009530534, ISSN: 0959-4973, DOI: 10.1097/00001813-200504000-00007
- KUO PO-LIN, HSU YA-LING, LIN TA-CHEN, LIN LIANG-TZUNG, CHANG JIUNN-KAE, LIN CHUN-CHING: "Casuarinin from the bark of Terminalia arjuna induces apoptosis and cell cycle arrest in human breast adenocarcinoma MCF-7 cells", PLANTA MEDICA, vol. 71, no. 3, 2005, pages 237 - 243, XP055848695, ISSN: 0032-0943, DOI: 10.1055/s-2005-837823
- V. BRINDHA , A. SARAVANAN , R. MANIMEKALAI: "Drug designing for ring finger protein 110 involved in adenocarcinoma (human breast cancer) using casuarinin extracted from Terminalia arjuna", INDIAN JOURNAL OF SCIENCE AND TECHNOLOGY, vol. 2, no. 2, 1 February 2009 (2009-02-01), IN, pages 22 - 26, XP009530535, ISSN: 0974-6846, DOI: 10.17485/ijst/2009/v2i2.10
- KASHIWADA, Y: "Antitumor agents, 129. Tannins and related compounds as selective cytotoxic agents", JOURNAL OF NATURAL PRODUCTS, vol. 55, no. 8, 1 August 1992 (1992-08-01), US, pages 1033 - 1043, XP009064352, ISSN: 0163-3864, DOI: 10.1021/np50086a002
- ANTONIO GONYALEY-SARRIAS , VERONICA MIGUEL: "The Gut Microbiota Ellagic Acid-Derived Metabolite Urolithin A and Its Sulfate Conjugate Are Substrates for the Drug Efflux Transporter Breast Cancer Resistance Protein (ABCG2/BCRP", JOURNAL OF AGRICULTURAL AND FOOD CHEMISTRY, vol. 61, no. 18, 15 April 2013 (2013-04-15), pages 4352 - 4359, XP002768228
- FERNANDES ANA, FERNANDES IVA, CRUZ LUÍS, MATEUS NUNO, CABRAL MIGUEL, DE FREITAS VICTOR: "Antioxidant and Biological Properties of Bioactive Phenolic Compounds from Quercus suber L", JOURNAL OF AGRICULTURAL AND FOOD CHEMISTRY, vol. 57, no. 23, 9 December 2009 (2009-12-09), US, pages 11154 - 11160, XP055848701, ISSN: 0021-8561, DOI: 10.1021/jf902093m
- WILLEMANN JESSYCA RIBEIRO, ESCHER GRAZIELA BRAGUETO, KANESHIMA TAI, FURTADO MARIANNA MIRANDA, SANT'ANA ANDERSON S., VIEIRA DO CARM: "Response surface optimization of phenolic compounds extraction from camu-camu (Myrciaria dubia) seed coat based on chemical properties and bioactivity", JOURNAL OF FOOD SCIENCE, vol. 85, no. 8, 1 August 2020 (2020-08-01), US, pages 2358 - 2367, XP055848703, ISSN: 0022-1147, DOI: 10.1111/1750-3841.15327

## Description

### TECHNICAL FIELD

The present invention generally relates to the field of cancer, and more particularly to castalagin for use in the treatment of cancers in combination with immune checkpoint inhibitors.

### BACKGROUND ART

The prevalence of cancer in human and animal populations and its role in mortality means there is a continuing need for new drugs which are effective against tumors. Elimination of a tumour or a reduction in its size or reducing the number of cancer cells circulating in the blood or lymph node systems may be beneficial in a variety of ways; reducing pain or discomfort, preventing metastasis, facilitating operative intervention, and more importantly prolonging life.

Various attempts have been made to help the immune system to fight tumors. One early approach, in the late 19^{th} century, involved a general stimulation of the immune system, e.g., through the administration of bacteria (live or killed) to elicit a general immune response which would also be directed against the tumor.

Recent approaches aimed at helping the immune system specifically to recognize tumorspecific antigens (TSAs) (or tumor associated antigens, TAAs) involve administration of tumorspecific antigens, typically combined with an adjuvant to the subject. However, a lack of a powerful immune response to TAAs is often observed in cancer. One of the factors responsible for the weak response to TAAs is the induction of inhibitory pathways/signals that suppress the immune response (often referred to as "immune checkpoints"). Whereas such inhibitory signals are important for maintenance of self-tolerance and to protect tissues from damage when the immune system is responding to pathogenic infection, they may also reduce what could otherwise be a helpful response by the body to the development of tumors.

A novel therapeutic era has come of age with immune checkpoint inhibitors or blockers (ICB) targeting inhibitory T-cell receptors such as CTLA-4, PD-L1 and PD-1 (Marabelle, Oncolmmunology 2016). This burgeoning field has even been awarded by the 2018 Nobel Prize in Medicine. These immunotherapeutic agents provide unparalleled clinical results in several advanced cancers including lung (Reck, NEJM 2016), melanoma (Robert, NEJM 2011), genitourinary (Motzer, NEJM 2018) as well as head and neck (Ferris, NEJM 2016). However, primary resistance rates range from 35-44% in patients with non-small cell lung cancer (NSCLC) while secondary resistance rates approach 100% (Reck, NEJM 2016).

Therefore, there is a need for the development of novel approaches to boost the response to ICB, and more particularly in cancers resistant to ICB therapy.

Auzanneau et (2012, Molecular Pharmacology, 82, 134-141) describes vescalagin for use in the treatment of cancer. Perchellet et al (Chemistry, 1994, 546, 303-327) describes the inhibitory activity of castalagin on 12-O-tetradecanoylphorbol-13-acetate- induced skin papillomas and carcinomas in mouse epidermis. Routy et al (2018, Science, 359, 91-97) describes a correlation between clinical responses of cancer patients to PD-1 immune checkpoint inhibitor therapy and the composition of the gut microbiota.

The present description refers to a number of documents, the content of which is herein incorporated by reference in their entirety.

### SUMMARY

The invention is set out in the appended set of claims.

The present application relates to :
- Castalagin for use in treating a subject suffering from a cancer resistant to immunotherapy, such as immune checkpoint inhibitor therapy.

Optionally, the immune checkpoint inhibitor is a Programmed cell death-1 (PD-1) inhibitor, a cytotoxic T-lymphocyte-associated antigen 4 (CTLA-4) inhibitor, or a Programmed death-ligand 1 (PD-L1) inhibitor.

Optionally, the immune checkpoint inhibitor is a blocking antibody.

Optionally, the immune checkpoint inhibitor is a PD-1 inhibitor.

Optionally, the castalagin is present in a plant or fruit extract, in particular a *Myrciaria dubia* extract.

Optionally, the castalagin is present in a pharmaceutical composition.

Optionally, the extract or pharmaceutical composition is formulated for delivery of the castalagin into the intestines, especially is formulated as a capsule.

Optionally, the cancer is lung cancer or breast cancer. Optionally, the lung cancer is non-small cell lung cancer (NSCLC). Optionally, the breast cancer is triple-negative breast cancer (TNBC).
- Castalagin for use in enhancing the anti-tumor immune response in a subject.

Optionally, the castalagin is for use in combination with an immune checkpoint inhibitor.

Optionally, the immune checkpoint inhibitor is a Programmed cell death-1 (PD-1) inhibitor, a cytotoxic T-lymphocyte-associated antigen 4 (CTLA-4) inhibitor, or a Programmed death-ligand 1 (PD-L1) inhibitor.

Optionally, the immune checkpoint inhibitor is a blocking antibody.

Optionally, the immune checkpoint inhibitor is a PD-1 inhibitor.

Optionally, the castalagin is present in a plant or fruit extract, especially a *Myrciaria dubia* extract.

Optionally, the castalagin is present in a pharmaceutical composition.

Optionally, the extract or pharmaceutical composition is formulated for delivery of the castalagin into the intestines, especially is formulated as a capsule.

Optionally, the subject suffers from skin cancer (*e.g*., melanoma, squamous cell skin cancer), lung cancer, renal cancer (*e.g*., renal cell carcinoma), Hodgkin lymphoma, head and neck cancer, colon cancer, liver cancer, stomach cancer, or myeloma, preferably lung cancer or breast cancer.

Optionally, the subject suffers from lung cancer, preferably non-small cell lung cancer (NSCLC).

Optionally, the subject suffers from breast cancer, preferably triple-negative breast cancer (TNBC).

### BRIEF DESCRIPTION OF DRAWINGS

In the appended drawings:
**FIG. 1A** is a schematic of the protocol used to study the effect of a Camu camu extract (CC) alone and additive effect when combined to anti-PD-1 therapy in a murine tumor model sensitive to anti-PD-1 therapy. Syngeneic C57BL/6 mice were implanted with 0.8 × 10⁶ MCA-205 sarcoma, subcutaneously and treated intraperitoneally (i.p.) when tumors reached 20 to 35 mm² in size with anti-PD-1 mAb (250µg/mouse; clone RMP1-14,) or isotype control (clone 2A3) with or without daily oral gavage with 200 mg/kg of CC (Camu camu Powder from SunFood).
**FIG. 1B** is graph showing the tumor size over time in the mice implanted with MCA-205 tumors treated with anti-PD-1 mAb or isotype control with or without daily oral gavage with CC.
**FIG. 1C****.** is a graph showing the tumor size at the sacrifice the mice implanted with MCA-205 tumors treated with anti-PD-1 mAb or isotype control with or without daily oral gavage with CC.
**FIG. 2A** is a schematic of the protocol used to study the effect of a Camu camu extract (CC) alone and additive effect when combine to anti-PD-1 therapy in a murine tumor model resistant to anti-PD-1 therapy. Syngeneic C57BL/6 mice were implanted with 0.5 × 10⁶ E0771 breast cancer tumor model subcutaneously and treated intraperitoneally (i.p.) when tumors reached 20 to 35 mm² in size with anti-PD-1 mAb (250µg/mouse; clone RMP1-14,) or isotype control (clone 2A3) with or without daily oral gavage with 200 mg/kg of CC (SunFood).
**FIG. 2B** is a graph showing the tumor size over time in the mice implanted with E0771 tumors after sequential injections of anti-PD-1 mAb (αPD-1) or isotype control (IsoPD-1) and daily oral gavage with water or CC.
**FIG. 2C** is a graph showing the tumor size at the sacrifice the mice implanted with E0771 tumors treated with anti-PD-1 mAb or isotype control with or without daily oral gavage with CC.

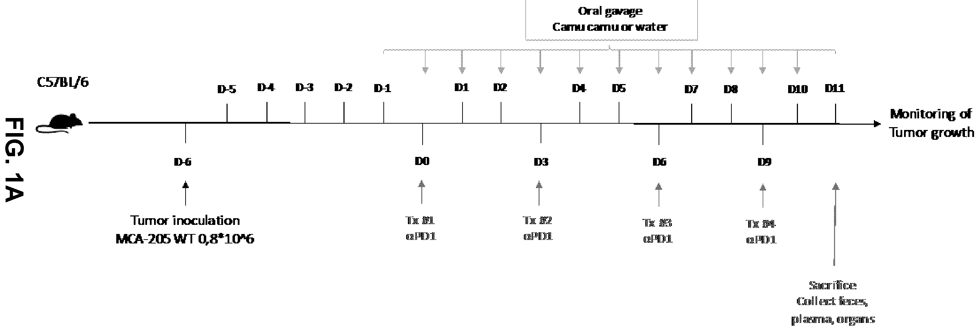

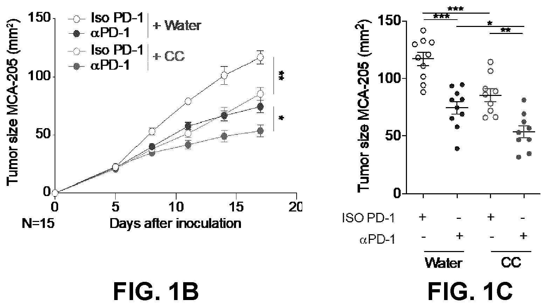

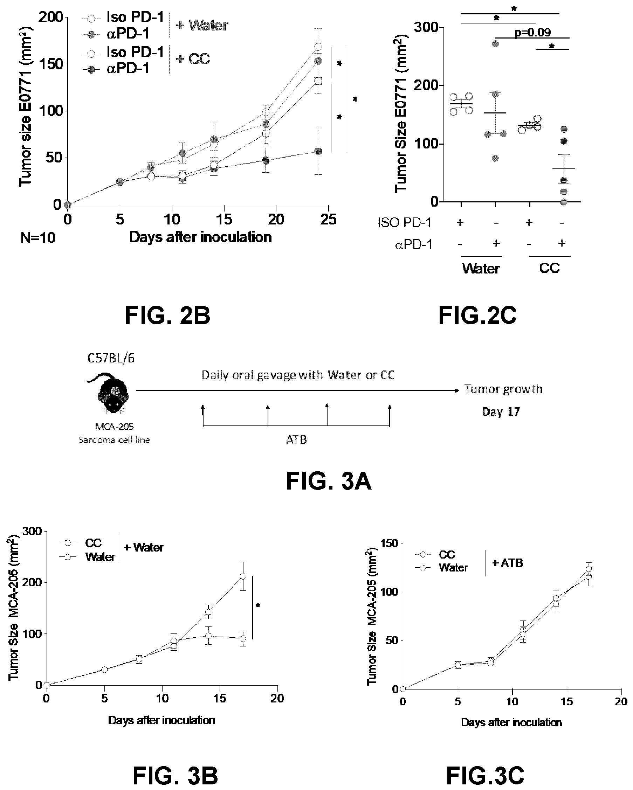

**FIG. 3A** is a schematic of the protocol used to study the effect of broad spectrum antibiotics (ATB) on the response to CC in the murine MCA-205 tumor model. Mice were treated with ATB 2 weeks before tumor implantation and continued on antibiotics until the end of the experiment. A mix of ampicillin (1 mg/ml), streptomycin (5 mg/ml), and colistin (1 mg/ml) (Sigma-Aldrich) were added in sterile drinking water. Solutions and bottles were changed 3 times a week. Antibiotic activity was confirmed by macroscopic changes observed at the level of caecum at the sacrifice (dilatation) and by cultivating the fecal pellets resuspended in sterile NaCl on blood agar plates for 48h at 37°C in aerobic or anaerobic conditions. MCA-205 inoculation and CC treatment were performed as in **FIG. 1A****.**
**FIGs. 3B-C** are graphs showing the tumor size over time in the mice implanted with MCA-205 tumors with or without daily oral gavage with CC, administered with water (control, **FIG. 3B****)** or ATB **(****FIG. 3C****)** (5 mice/group).
**FIG. 4A** is a schematic of the protocol used to study the effects of fecal microbiota transfer (FMT) from CC-treated mice on the response to anti-PD-1 in the murine MCA-205 tumor model. Feces from mice treated with CC were frozen in Eppendorf^{®} tubes at -80°C. MCA-205 was implanted subcutaneously and treated intraperitoneally (*i.p*.) when tumors reached 20 to 35 mm² in size with anti-PD-1 mAb (250µg/mouse; clone RMP1-14,) or isotype control (clone 2A3) with or without daily oral gavage with diluted stool in NaCl. 100µg of feces were resuspended in 1mL of sterile NaCl.
**FIG. 4B** is a graph showing the tumor size at the sacrifice in the mice implanted with MCA-205 tumors treated with anti-PD-1 mAb or isotype control with or without daily oral gavage with diluted stool in NaCl.
**FIG. 5A** is a schematic of the experimental design of avatar mice experiments. FMT from feces samples from Non-responders (NR) and responders (R) Non-small cell lung cancer (NSCLC) patients were individually performed after 3 days of ATB in SPF C57BI6 mice. Two weeks later, MCA-205 sarcoma cells were inoculated and daily gavage with water or CC were performed in combination with sequential injections of αPD-1 or IsoPD-1 mAb.
**FIG. 5B** is a graph showing pooled means tumor +/- SEM at the sacrifice (D+17) post FMT from 2 NR and 2 R groups for each CC and water groups.
**FIG. 5C** is a graph showing the representation of the number of observed genus for the *alpha*-diversity of R and NR at baseline (before CC gavage and after 14 days of engraftment in MCA-205-bearing, SPF-reared mice (n=10) treated with ATB and then receiving FMT from 4 NSCLC patients (n = 2 NR, n = 2 R). Means ± SEM are represented.
**FIG. 5D** is a Bray-Curtis representation of the *beta*-diversity of the 16s RNA sequencing after 2 weeks of engraftment of NR and R FMT at the genus level. **p <* 0.05, ****p <* 0.001.
**FIG. 5E** is a Volcano plot representation of differential abundance analysis results after 16s sequencing analysis of mouse feces after 14 days received NR or R FMT Day 0.
**FIG. 5F** is a graph showing the *alpha*-diversity represented by observed genus in NR and R FMT groups at D+11. Means ± *SEM* are represented. **p* < 0.05, ***p <* 0.01.
**FIG. 6A** is a graph showing the 16s rRNA fecal samples from mice from the four groups in the MCA-205 experiments **(****FIG. 1A****)** and representation of the *alpha*-diversity measured by the Shannon index in each group.
**FIG. 6B** is a graph showing the results of real-time PCR assays on DNA extracted from mouse feces after 6 days of water or CC gavage using specific primers for 16s detection in the MCA-205 model (n=10 mice/group).
**FIG. 6C** is a graph showing the beta-diversity measured by Bray-Curtis Index comparing baseline (pre-treatment) with CC or water pooled (αPD1 and IsoPD-1) groups.
**FIG. 6D** is a graph showing the 16s rRNA microbiome profiling of samples from MCA-205 experiments **(****FIG. 1A****)** and representation of Beta-diversity measured by Bray-Curtis Index comparing all four groups after 6 days of treatment.
**FIG. 6E** is a Volcano plot representation of differential abundance analysis comparing pooled CC versus water groups in the MCA-205 tumor. Bacteria enriched in each group are represented using adjusted p-value and p-value. ***p* < 0.01.
**FIG. 6F** is a Volcano plots representation of differential abundance analysis in the Water/IsoPD-1 vs CC/IsoPD-1 groups in the MCA-205 tumor model. Bacteria enriched in each group are represented using adjusted p-value and p-value. ***p* < 0.01.
**FIG. 6G** is a Volcano plots representation of differential abundance analysis in the Water/IsoPD-1 vs CC/αPD-1 groups in the MCA-205 tumor model. Bacteria enriched in each group are represented using adjusted p-value and p-value. ***p* < 0.01.
**FIG. 6H** is a Volcano plot representation of differential abundance analysis comparing the water/αPD-1 *versus* CC/αPD1 groups in the E0771 tumor. Bacteria enriched in each group are represented using adjusted p-value and p-value (FDR:0.1). **p* < 0.05, ***p <* 0.01, ****p* < 0.001.
**FIG. 6I** is a Volcano plots representation of differential abundance analysis in the Water/IsoPD-1 vs CC/IsoPD-1 group in the E0771 model.

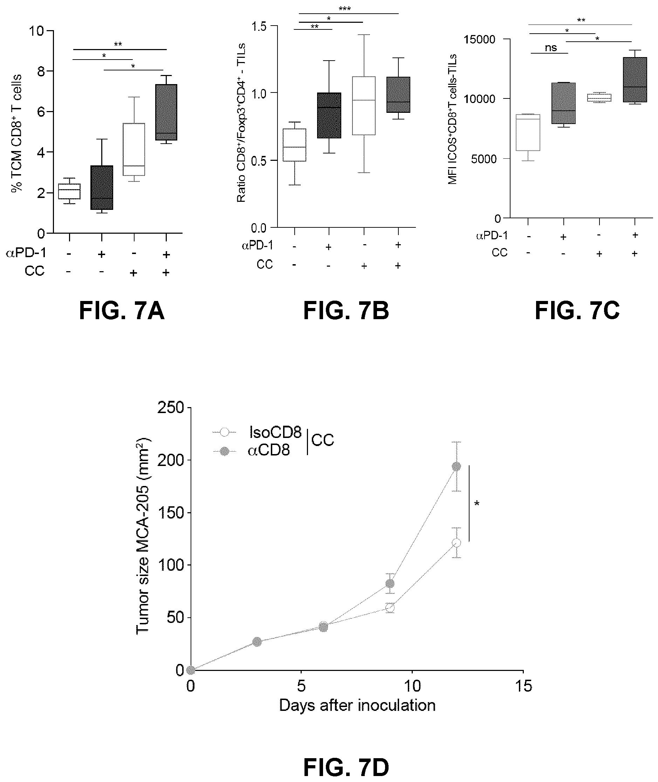

**FIGs. 7A-C** are graphs showing the results of immune cell profiling by flow cytometry in the murine MCA-205 **(****FIGs. 7A-B)** or E0771 **(****FIG. 7C****)** tumor model treated with anti-PD-1 and/or CC. The tumors and the spleens were harvested 9 days or 19 days after the first injection of anti-PD-1 mAb into mice bearing MCA-205 or E0771 tumors, respectively. Excised tumors were cut into small pieces and digested in RPMI medium containing Liberase 25 µg/mL (Roche) and DNase1 at 150 UI/mL (Roche) for 30 minutes at 37°C and then crushed and filtered twice using 100 and 70 µm cell strainers (Becton & Dickinson). Spleens were crushed in RPMI medium and subsequently filtered through a 100 µm cell strainer. Two million tumor cells or splenocytes were pre-incubated with purified anti-mouse CD16/CD32 (clone 93; eBioscience) for 30 minutes at 4°C, before membrane staining (CD45, CD3, CD4, CD8, PD1, PDL1, ICOS, CXCR3, CCR9, CD45RB, CD62L, CD44). For intracellular staining, the Foxp3 staining kit (eBioscience) was used. Dead cells were excluded using the Live/Dead Fixable aqua blue dead cell stain kit (Life Technologies). **FIG. 7A-B** is a graph showing TCM CD8⁺ T cells (CD45RB⁻CD62L⁺CD8⁺ T cells) and the ratio CD8⁺ T cells/Foxp3+CD4+ T cells (Treg) respectively in TILs from mice with MCA-205 tumors following treatment with anti-PD-1 mAb or isotype control with or without daily oral gavage with CC. **FIG. 7C** is a graph showing the activation of intratumoral CD8⁺T cells (as assessed by MFI of ICOS⁺CD8⁺T cells by flow cytometry) in TILs in the E0771 tumor model post-treatment with CC +/- αPD-1.
**FIG. 7D** is a graph showing the effects of blocking CD8⁺ T cell activity on the antitumor effect of CC in the murine MCA-205 tumor model. Syngeneic C57BL/6 mice were implanted with 0.8 × 10⁶ MCA-205 sarcoma, subcutaneously and 3 days after the tumor inoculation the mice were treated with 150 µg/mouse of an anti-CD8 (clone: 53-5.8, BioXCell) or isotype control. Then, when the tumors reached 20 to 35 mm² in size, the mice were administered or not a daily oral gavage with 200 mg/kg of CC.
**FIG. 7E** is a pairwise Spearman rank correlation heatmap between significantly different bacteria enriched in CC/isoPD-1 vs water/isoPD-1 group (n=1) with positively correlated TILs cytometry and matching tumor size in the MCA-205 experiment. Left panel within the TILs and right panel the splenocytes.
**FIG. 7F** is pairwise Spearman rank correlation heatmap between significantly different fecal taxa enriched in CC/αPD-1 vs water/αPD-1 group and frequency of indicated cell types by flow cytometry and matching tumor size in the E0771 tumor model. Unpaired t-tests were used. **p <* 0.05, ***p* < 0.01, ****p* < 0.001.
**FIG. 8A** shows the fractionation workflow diagram of the CC extract.
**FIG. 8B** is a diagram of the high-performance liquid chromatography retention time of the complete Camu-camu extraction, followed by HPLD retention time in the polar fraction and fraction P3 as well as castalagin extracted from oak.
**FIG. 8C** is a graph showing the effect of the various fractions depicted in **FIG. 7A** (P, NP, M, INS) in the presence or not of anti-PD-1 in MCA-205-bearing, SPF-reared mice (n=5, mean +/- SEM tumor sizes at sacrifice). Using the same experimental design as described previously **(****FIG. 1A****),** mice were administered or not with a daily oral gavage with each fraction (Polar fraction, P; Non-polar fraction, NP; medium polarity, M; and insoluble fraction, INS) at a concentration of 40.18 mg/kg or with CC at the dose of 100 mg/kg. Unpaired t-tests were used. **p* < 0.05, ***p* < 0.01.
**FIG. 8D** is a graph showing the effects of different subfractions (P1, P2, P3, P4) from fraction P of **FIG. 7C** in the presence or absence of anti-PD-1 in the murine MCA-205 tumor model. Using the same experimental design as described previously **(****FIG. 1A****),** mice were administered or not with a daily oral gavage with each fraction (P1, P2, P3 and P4) at the concentration of 0.85 mg/kg or with CC at the dose of 100 mg/kg. Unpaired t-tests were used. **p* < 0.05, ***p* < 0.01.
**FIG. 8E** is a graph showing the effects of different dosages of castalagin in the presence of anti-PD-1 in the murine MCA-205 tumor model (mean MCA-205 tumor sizes represented at the sacrifice of mic. Using the same experimental design as described previously **(****FIG. 1A****),** mice were administered or not with a daily oral gavage with increasing doses of castalagin (from 0.11 mg/kg to 2.56 mg/kg) or with CC at the dose of 100 mg/kg. Of note, the dose present in CC is equivalent to about 0.85 mg/kg. For the negative control at 0 mg/kg, mice received water.
**FIG. 8F** is a graph showing the effects of castalagin in the presence of anti-PD-1 in E0771-bearing, SPF-reared mice (n=5, tumor sizes at sacrifice). Using the same experimental design as described previously **(****FIG. 2A****),** mice were administered or not with a daily oral gavage with castalagin (0.85 mg/kg per mouse) in the presence of not of anti-PD-1.
**FIG. 9A** is a graph showing the effects of castalagin administration at the standard concentration (0.85 mg/kg per mouse) in germ-free conditions on tumor size in the murine MCA-205 tumor model.
**FIG. 9B** is a graph showing the bacterial diversity (# of observed genus) at day 5 (baseline) and at Day11 after castalagin gavage in the MCA-205 model. Unpaired t-tests were used. **p*< 0.05, ***p* < 0.01, ****p* < 0.001.
**FIG. 9C** is a Bray-Curtis beta-diversity representation from 16s rRNA microbiome sequencing at day Castalagin or water gavage in MCA-205-bearing, SPF-reared mice (n=5). Each line corresponds to a mouse group and each dot corresponds to one animal. Unpaired t-tests were used. **p* < 0.05, ***p* < 0.01, ****p* < 0.001.
**FIG. 9D** is a Volcano plot representation of differential abundance analysis results after 16s sequencing analysis in the water/IsoPD-1 versus castalagin/IsoPD-1 group in MCA-205-bearing, SPF-reared mice (n=5).
**FIGs. 9E-I** show relative abundance analysis of *Ruminococcus, Alistepes, Christensenellaceae R7 group, Paraprevotella and Lachnoclostridium* results after 16s sequencing analysis between water and castalagin groups in the NR FMT experiment. **p* < 0.05, ***p <* 0.01, ****p* < 0.001.
**FIG. 9J** is a graph showing the effect of castalagin treatment (0 mg/kg, 1/4X, 0.21 mg/kg, 1X, 0.85 mg/kg, and 3X, 2.55 mg/kg) on the amount of *Ruminococcaceae* in the feces. Real-time PCR was performed on DNA extracted from mouse stools after 6 days of oral gavage with water or castalagin at 0.21 mg/kg, 0.85 mg/kg and 2.55 mg/kg using specific primers for Ruminococcaceae detection in MCA-205-bearing, SPF-reared mice (n=5). **p* < 0.05, ***p* < 0.01.
**FIGs. 10A** are graphs showing the results of the effects of castalagin treatment on the immune cell profile in the murine MCA-205. Flow cytometry analysis of MCA-205 TILs of CD8⁺T Central Memory (TCM) cells (CD45RB⁻CD62L⁺) in the germ-free and SPF experiments comparing CC versus water at the sacrifice.
**FIG. 10B** Representative images for the CD4, CD8 and Foxp3 immunofluorescence staining of tumors in both water/ISoPD-1 and castalagin/IsoPD-1 groups.
**FIG. 10C** Box plot for the ratio CD8⁺/Foxp3⁺CD4⁺ in tumor in (n=8/group) obtained with immunofluorescence staining. **p* < 0.05.
**FIG. 10D-E** are graphs showing the results of the effects of castalagin treatment on the immune cell profile in the murine E0771. Flow cytometry analysis of E0771 of memory CD8⁺ T cells in tumor **(****FIG. 10D****)** and in spleen **(****FIG. 10E****)** at the sacrifice.
**FIG. 11A** is a graph showing the effect of castalagin (0.85 mg/kg) in the presence or not of anti-PD-1 on tumor growth kinetics in the in the ATB-avatar model after FMT from 1 NR NSCLC patient with daily gavage with castalagin or water in combination with αPD1 mAb or IsoPD-1. Unpaired t-tests were used. Means ± SEM are represented. **p* < 0.05.
**FIG. 11B** is a graph showing the therapeutic effect of castalagin post-FMT using fecal samples from NR NSCLC patients in ATB and germ-free conditions. Fecal microbiota transplantation (FMT) of stool sample from a Non-responders (NR) Non-small cell lung cancer (NSCLC) patient (n= 1NR) was performed in germ-free C57BL/6 mice (n=3). Two weeks later, MCA-205 sarcoma cells were inoculated, and a daily gavage with water or castalagin were performed. Each line corresponds to a mouse group and each dot corresponds to one animal. Unpaired t-tests were used. Means ± SEM are represented.
**FIG. 12A** is a schematic of the hydrolysis of castalagin into ellagic acid and castalin, and of the metabolism of ellagic acid into urolithins by the gut microbiome.
**FIG. 12B** is a graph showing the effect of castalagin, vescalagin, ellagic acid, castalin and urothelin A on tumor size at the sacrifice in the murine MCA-205 tumor model using the same experimental design as described previously **(****FIG. 1A****).**
**FIG. 12C** is a diagram depicting the *in vitro* labelling of castalagin with fluorescein.
**FIG. 12D** is a representation of one flow cytometry experiment representation of fluorescein-labelled castalagin in co-culture with *Escherichia Coli, Ruminococcus bromii* and *Bacteroides thetaiotomicron.* The upper panels represent the unstained conditions and the lower panels represent the staining with fluorescein-castalagin at 37°C.
**FIG. 12E** is a graph showing the results of competitive assay of *R. bromii* and *E. Coli* in the presence of fluorescein bound castalagin at 37°C and 0°C and in the presence of unbound castalagin at 100 x concentration. Each dot represents one experiment.
**FIG. 12F** representation images by epifluorescence inversed microscopy of *R. bromii, E. coli, B. thetaiotaomicron* after fluo-castalagin.
**FIG. 12G-H.** are graphs depicting the results of qPCR assays of diversity (16s) and Ruminococcaceae in two non-cancer HIV patients treated with daily 1.5 mg of CC. The graphs depict the diversity (16s RNA) **(****FIG. 12G****)** and amount of Ruminococcaceae DNA **(****FIG. 12H****)** before CC administration and 3 weeks later.
**FIG. 13** is a graph depicting the results of qPCR assays performed on DNA extracted from mouse stools after 6 days of oral gavage with water or castalagin at 0.85 mg/kg using specific primers for Ruminococcaceae detection in MCA-205-bearing, SPF-reared mice (n=10). **p <* 0.05,

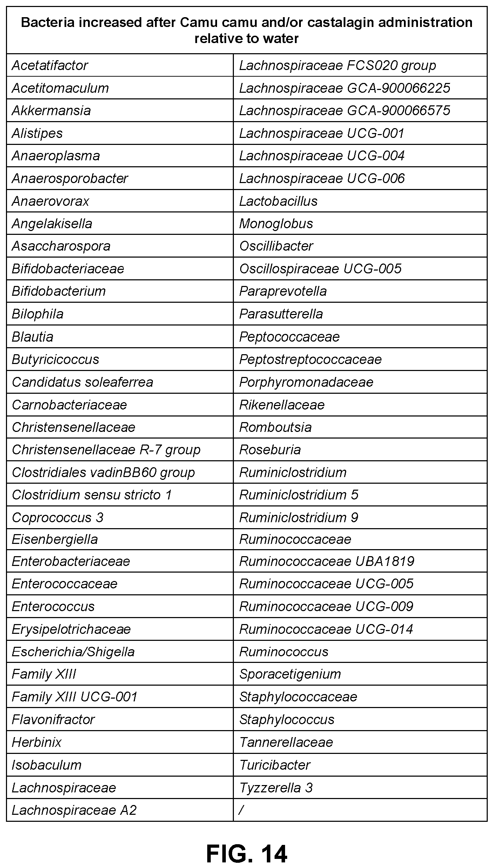

**FIG. 14** is a Table showing the list of Bacteria increased after CC and/or castalagin administration compared to water.

### DETAILED DISCLOSURE

The use of the terms "a" and "an" and "the" and similar referents in the context of describing the invention (especially in the context of the following claims) are to be construed to cover both the singular and the plural, unless otherwise indicated herein or clearly contradicted by context.

The terms "comprising", "having", "including", and "containing" are to be construed as open-ended terms (i.e., meaning "including, but not limited to") unless otherwise noted.

Recitation of ranges of values herein are merely intended to serve as a shorthand method of referring individually to each separate value falling within the range, unless otherwise indicated herein, and each separate value is incorporated into the specification as if it were individually recited herein. All subsets of values within the ranges are also incorporated into the specification as if they were individually recited herein.

All methods described herein can be performed in any suitable order unless otherwise indicated herein or otherwise clearly contradicted by context.

The use of any and all examples, or exemplary language ("*e.g*.", "such as", etc.) provided herein, is intended merely to better illustrate the invention.

Herein, the term "about" has its ordinary meaning. The term "about" is used to indicate that a value includes an inherent variation of error for the device or the method being employed to determine the value, or encompass values close to the recited values, for example within 10% of the recited values (or range of values).

Unless otherwise defined, all technical and scientific terms used herein have the same meaning as commonly understood by one of ordinary skill in the art to which this invention belongs.

Any and all combinations and subcombinations of the aspects and features disclosed herein are encompassed by the present disclosure.

In the studies described herein, the present inventors have demonstrated in two murine tumor models that a crude extract from *Myrciaria dubia* (Camu camu, CC) berries was able to induce an antitumor response and to enhance the antitumor response of immune checkpoint inhibitors, but also to restore the antitumor response of immune checkpoint inhibitors in resistant tumors. The present inventors have also provided compelling evidence that the effect of the Camu camu extract was mediated at least in part by modulation of the gut microbiota, and involves the T-cell-mediated immune response. Further characterization of the Camu camu extract has led to the identification of castalagin as the main active ingredient responsible for the effect of the extract on the antitumor response.

Accordingly, in a first aspect, the present disclosure provides castalagin for use in inducing or restoring a response to an immunotherapy, such as an immune checkpoint inhibitor therapy, in a subject suffering from a cancer resistant to such immunotherapy (*e.g*., immune checkpoint inhibitor therapy).

In another aspect, the present disclosure provides s a combination therapy comprising castalagin in combination with an immunotherapy (*e.g*., immune checkpoint inhibitor) for treating a subject suffering from a cancer resistant to such immunotherapy (*e.g*., immune checkpoint inhibitor therapy).

In another aspect, the present disclosure provides castalagin for use in enhancing the immune response, such as the anti-tumor immune response, in a subject.

In an aspect, the above-noted treatment increases the levels of immune cells such as T cells in the tumor (*e.g*., tumor-infiltration lymphocytes or TILs). In an aspect, the T cells are CD4⁺ and/or CD8⁺ T cells such as activated (ICOS⁺) and/or memory CD4⁺ and/or CD8⁺ T cells (central memory (T_{CM}) CD4⁺ and/or CD8⁺ cells). In another aspect, the above-noted treatment increases the CD8⁺ T cells/Foxp3⁺CD4⁺T cells (Treg) ratio. In another aspect, the above-noted treatment increases the ICOS⁺Foxp3⁻CD4⁺ T cells.

The present disclosure also provides castalagin for use in increasing the levels of bacteria of the family or genus *Acetatifactor, Lachnospiraceae FCS020 group, Acetitomaculum Lachnospiraceae GCA-900066225, Akkermansia, Lachnospiraceae GCA-900066575, Alistipes, Lachnospiraceae UCG-001, Anaeroplasma, Lachnospiraceae UCG-004, Anaerosporobacter, Lachnospiraceae UCG-006, Anaerovorax, Lactobacillus, Angelakisella Monoglobus, Asaccharospora, Oscillibacter, Bifidobacteriaceae, Oscillospiraceae UCG-005, Bifidobacterium, Paraprevotella, Bilophila, Parasutterella, Blautia, Peptococcaceae, Butyricicoccus, Peptostreptococcaceae, Candidatus soleaferrea, Porphyromonadaceae, Carnobacteriaceae, Rikenellaceae, Christensenellaceae, Romboutsia, Christensenellaceae R-7 group, Roseburia, Clostridiales vadinBB60 group, Ruminiclostridium, Clostridium sensu stricto 1, Ruminiclostridium 5, Coprococcus 3, Ruminiclostridium 9, Eisenbergiella, Ruminococcaceae, Enterobacteriaceae, Ruminococcaceae UBA* 1819, *Enterococcaceae, Ruminococcaceae UCG-005, Enterococcus, Ruminococcaceae UCG-009, Erysipelotrichaceae, Ruminococcaceae UCG-014, Escherichia*/*Shigella, Ruminococcus, Family XIII, Sporacetigenium, Family XIII UCG-001, Staphylococcaceae, Flavonifractor, Staphylococcus, Herbinix, Tannerellaceae, Isobaculum, Turicibacter, Lachnospiraceae, Tyzzerella 3 and*/*or Lachnospiraceae A2,* preferably *Turicibacter, Bilophila, Ruminococcaceae (e.g., Ruminococcaceae UBA 1819), Parasutterella, Clostridium sensu stricto 1, Chrinstensenellaceae, Alistipes, Ruminococcus, Akkermansia,* and/or *Anaeroplasma,* in the intestines of a subject.

In an aspect, the above-mentioned use increases the levels of bacteria of the family or genus *Turicibacter.* In an aspect, the above-mentioned use increases the levels of bacteria of the family or genus *Bilophila.* In an aspect, the above-mentioned use increases the levels of bacteria of the family or genus *Ruminococcaceae (e.g., Ruminococcaceae UBA1819).* In an aspect, the above-mentioned use increases the levels of bacteria of the family or genus *Parasutterella.* In an aspect, the above-mentioned use increases the levels of bacteria of the family or genus *Clostridium sensu stricto* 1. In an aspect, the above-mentioned use increases the levels of bacteria of the family or genus *Akkermansia.* In an aspect, the above-mentioned use increases the levels of bacteria of the family or genus *Anaeroplasma.* In a further aspect, the bacteria of the family or genus *Akkermansia* is *Akkermensia muciniphilia.*

In another aspect, the present disclosure provides castalagin for use in decreasing the levels of bacteria of the family or genus *Lactobacillus* and/or *Pseudoflavonifractor* in the intestines of a subject.

The present disclosure also provides a combination therapy comprising castalagin and an immunotherapy, such as an immune checkpoint inhibitor. The present disclosure also provides the use of a combination therapy comprising castalagin and an immunotherapy (i.e. immunotherapeutic agent), such as an immune checkpoint inhibitor, for treating a subject suffering from a cancer (*e.g*., a cancer resistant to immunotherapy such as immune checkpoint inhibitor monotherapy).

Castalagin (molecular weight 934.63, CAS No. 24312-00-3) has the following structure:

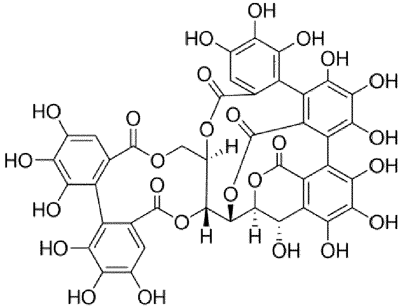

It is the (33beta) isomer of vescalagin (molecular weight 934.63, CAS No. 36001-47-5), which has the following structure:

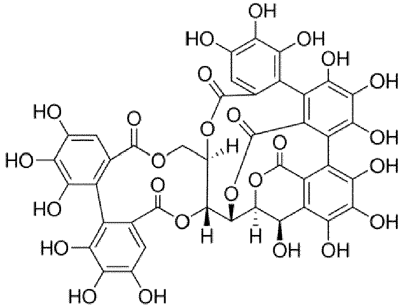

Castalagin and vescalagin belong to a particular group of ellagitannins that are composed of a series of highly hydrosoluble C-glucosidic variants.

Thus, analogs of castalagin include castalagin glycosides such as grandinin (lyxose) and roburin E (xylose), casuarinin, and castalin. The castalagin analog may also be ethoxylated castalagin as described in WO2014/071438. The castalagin analog retain or share the biological activity of castalagin, and more particularly the ability to improve the immune response (anti-tumor immune response) and to restore the response to an immunotherapy such as immune checkpoint inhibitor therapy in subjects. In an aspect, the castalagin analog is a castalagin salt, preferably a pharmaceutically acceptable salt. The term "salt(s)", as employed herein, denotes acidic salts formed with inorganic and/or organic acids, as well as basic salts formed with inorganic and/or organic bases. Salts for use in pharmaceutical compositions will be pharmaceutically acceptable salts. As used herein the term "pharmaceutically acceptable salt" refers to salts of castalagin that retain the biological activity of castalagin, and which are not biologically or otherwise undesirable.

For example, a salt of castalagin may be an acid addition salt, such as hydrochloric, hydrobromic, phosphoric, acetic, trifluoacetic, lactic, pyruvic, malonic, succinic, glutaric, fumaric, tartaric, maleic acid, citric, ascorbic, methane-or ethane-sulfonic acid, or camphoric. It may also be a base addition salt, such as sodium or potassium hydroxide, triethylamine or tert-butylamine. Such salts can be formed quite readily by those skilled in the art using standard techniques. Indeed, the chemical modification of a pharmaceutical compound (i.e. castalagin) into a salt is a technique well known to pharmaceutical chemists, (See, e.g., H. Ansel et. al., Pharmaceutical Dosage Forms and Drug Delivery Systems (6th Ed. 1995) at pp. 196 and 1456-1457; P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website). Salts of castalagin may be formed, for example, by reacting castalagin with an amount of acid or base, such as an equivalent amount, in a medium such as one in which the salt precipitates or in an aqueous medium followed by lyophilization.

Castalagin may be found or isolated from a variety of sources including fruits and/or plant extracts such as extracts from *Myrciaria dubia* (Camu-camu) berries, extracts of lythrum salicaria (see, *e.g*., WO/2016/102874), extracts from oak (Quercus sp.), extracts from chestnut (Castanea sp.), extracts from stem barks of *Anogeissus leiocarpus* and *Terminalia avicennoides* (Shuaibu MN et al., Parasitology Research. 103(6): 1333-8), extracts from the leaves of *Syzygium* samarangense (Blume) (Kamada et al., Fitoterapia, Volume 129, September 2018, Pages 94-101). Methods to isolate Castalagin and/or other C-glucosidic ellagitannins are well known in the art and are notably described in some of the above-noted references as well as in Araujo et al., Rsc Advances, 2015, 5, 96151-96157 and Stine et al., Methods in Molecular Biology (Clifton, N.J.), 2011, 670, 13-32).

For the use, and therapy described herein, the castalagin may be used in the form of an extract (fruits and/or plant extracts) comprising a suitable amount of castalagin or analog thereof, including a crude extract or a partially purified extract enriched in castalagin or analog thereof, or may be in purified form (either isolated from a natural source or synthesized). Thus, in an aspect, an extract comprising castalagin is used or administered. In another aspect, purified or isolated castalagin is used or administered. In an aspect, the purified or isolated castalagin
The skilled person would understand that the extract or the purified castalagin may be mixed with one or more carriers and/or excipients (pharmaceutically acceptable carriers and/or excipients) to obtain a composition suitable for administration to the subject.

An "excipient," as used herein, has its normal meaning in the art and is any ingredient that is not an active ingredient (drug) itself. Excipients include for example buffers, binders, lubricants, diluents, fillers, thickening agents, disintegrants, plasticizers, coatings, barrier layer formulations, stabilizing agent, release-delaying agents and other components. "Pharmaceutically acceptable excipient" as used herein refers to any excipient that does not interfere with effectiveness of the biological activity of the active ingredients and that is not toxic to the subject, *i.e.,* is a type of excipient and/or is for use in an amount which is not toxic to the subject. Excipients are well known in the art, and the present composition is not limited in these respects. The carrier/excipient can be suitable, for example, for intravenous, parenteral, subcutaneous, intramuscular, intracranial, intraorbital, ophthalmic, intraventricular, intracapsular, intraspinal, intrathecal, epidural, intracisternal, intraperitoneal, intranasal or pulmonary (*e.g*., aerosol) administration. Therapeutic compositions are prepared using standard methods known in the art by mixing the active ingredient having the desired degree of purity with one or more optional pharmaceutically acceptable carriers, excipients and/or stabilizers (see Remington: The Science and Practice of Pharmacy, by Loyd V Allen, Jr, 2012, 22nd edition, Pharmaceutical Press; Handbook of Pharmaceutical Excipients, by Rowe et al., 2012, 7th edition, Pharmaceutical Press).

In an aspect, the castalagin is formulated for oral administration. Formulations suitable for oral administration may include (a) liquid solutions, such as an effective amount of active agent(s)/composition(s) suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, *e.g*., sucrose, as well as pastilles comprising the active ingredient in an inert base, such as gelatin and glycerin or sucrose and acacia emulsions, gels, and the like containing, in addition to the active ingredient, carriers known in the art.

In an aspect, the castalagin is formulated for parenteral administration (*e.g*., injection). Formulations for parenteral administration may, for example, contain excipients, sterile water, or saline, polyalkylene glycols such as polyethylene glycol, oils of vegetable origin, or hydrogenated napthalenes. Biocompatible, biodegradable lactide polymer, lactide/glycolide copolymer, or polyoxyethylene-polyoxypropylene copolymers may be used to control the release of the compounds. Other potentially useful parenteral delivery systems castalagin include ethylenevinyl acetate copolymer particles, osmotic pumps, implantable infusion systems, and liposomes. Formulations for inhalation may contain excipients, (*e.g*., lactose) or may be aqueous solutions containing, for example, polyoxyethylene-9-lauryl ether, glycocholate and deoxycholate, or may be oily solutions for administration in the form of nasal drops, or as a gel.

In an aspect, the castalagin is formulated for enteric delivery, i.e. delivery into the intestines. This may be achieved by methods well known in the art. For example, the castalagin may be coated or encapsulated with an enteric agent or material. Enteric agents for instance allow release at certain pHs or in the presence of degradative enzymes or bacteria that are characteristically present in specific locations of the GI tract (*e.g*., small intestine, large intestine, or specific regions thereof) where release is desired. In an aspect, the enteric material is pH-sensitive and is affected by changes in pH encountered within the gastrointestinal tract (pH-sensitive release). The enteric material typically remains insoluble at gastric pH, then allows for release of the active ingredient in the higher pH environment of the downstream gastrointestinal tract (*e.g*., often the duodenum, or sometimes the colon). In another aspect, the enteric material comprises enzymatically degradable polymers that are degraded by bacterial enzymes (*e.g*., carbohydrate processing enzymes such as glycosidases, polysaccharide lyases and carbohydrate esterases) present in the lower gastrointestinal tract, particularly in the colon. Such enteric materials include, for example, cellulosic polymers such as hydroxypropyl cellulose, hydroxyethyl cellulose, hydroxymethyl cellulose, hydroxypropyl methyl cellulose, hydroxypropyl methyl cellulose acetate succinate, hydroxypropylmethyl cellulose phthalate, methylcellulose, ethyl cellulose, cellulose acetate, cellulose acetate phthalate, cellulose acetate trimellitate and carboxymethylcellulose sodium; acrylic acid polymers and copolymers, preferably formed from acrylic acid, methacrylic acid, methyl acrylate, ethyl acrylate, methyl methacrylate and/or ethyl methacrylate, and other methacrylic resins that are commercially available under the trade-name Acryl-EZE^{®} (Colorcon, USA), Eudragit^{®} (Rohm Pharma; Westerstadt, Germany), including Eudragit^{®} L30D-55 and L100-55 (soluble at pH 5.5 and above), Eudragit^{®} L-IOO (soluble at pH 6.0 and above), Eudragit^{®} S (soluble at pH 7.0 and above, as a result of a higher degree of esterification), and Eudragits^{®} NE, RL and RS (water-insoluble polymers having different degrees of permeability and expandability); vinyl polymers and copolymers such as polyvinyl pyrrolidone, vinyl acetate, vinylacetate phthalate, vinylacetate crotonic acid copolymer, and ethylene-vinyl acetate copolymer; enzymatically degradable polymers such as azo polymers, pectin, chitosan, amylose and guar gum; zein and shellac. Combinations of different enteric materials may also be used. Approaches for colon specific drug delivery are well known in the art (see, *e.g.,* Philip et al., Oman Med J. 2010 Apr; 25(2): 79-87; Lee et al., Pharmaceutics. 2020 Jan; 12(1): 68), and include pH-dependent systems (*e.g*., using pH-dependent polymers), receptor-mediated systems, magnetically-driven systems, delayed or time-dependent systems, microbially triggered drug delivery systems (*e.g*., comprising sugar-based polymers that may be degraded by enzymes produced by the colon microflora such as glucoronidase, xylosidase, arabinosidase, galactosidase), pressure controlled colonic delivery capsule (drug release induced by the higher pressures encountered in the colon), osmotic controlled drug delivery, as well as any combinations of these approaches (*e.g*., colon targeted delivery system (CODESTM) using a combined approach of pH dependent and microbially triggered drug delivery).

In an aspect, the castalagin is formulated in a capsule made of an enteric material (enteric capsule).

Any suitable amount of the castalagin may be administered to a subject. The dosages will depend on many factors including the mode of administration. Typically, the amount of castalagin contained within a single dose will be an amount that effectively prevent, delay or treat cancer without inducing significant toxicity.

For the prevention, treatment or reduction in the severity of a given disease or condition (cancer), the appropriate dosage of the castalagin will depend on the type of disease or condition to be treated, the severity and course of the disease or condition, whether the castalagin is administered for preventive or therapeutic purposes, previous therapy, the patient's clinical history and response to the castalagin or analog thereof, and the discretion of the attending physician. The castalagin is suitably administered to the patient at one time or over a series of treatments. Preferably, it is desirable to determine the dose-response curve *in vitro,* and then in useful animal models prior to testing in humans. The present disclosure provides dosages for the castalagin and compositions comprising same. For example, depending on the type and severity of the disease, about 1 µg/kg to to 1000 mg per kg (mg/kg) of body weight per day. Further, the effective dose may be 0.5 mg/kg, 1 mg/kg, 5 mg/kg, 10 mg/kg, 15 mg/kg, 20 mg/kg/ 25 mg/kg, 30 mg/kg, 35 mg/kg, 40 mg/kg, 45 mg/kg, 50 mg/kg, 55 mg/kg, 60 mg/kg, 70 mg/kg, 75 mg/kg, 80 mg/kg, 90 mg/kg, 100 mg/kg, 125 mg/kg, 150 mg/kg, 175 mg/kg, 200 mg/kg, and may increase by 25 mg/kg increments up to 1000 mg/kg, or may range between any two of the foregoing values. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs (*e.g*., reduction of tumor volume or tumor cell number). However, other dosage regimens may be useful. The progress of this therapy is easily monitored by conventional techniques and assays. In an aspect, the dosage for administration to a human subject corresponds to a dosage of at least 0.8 mg castalagin/kg in a mouse.

The term immunotherapy as used herein refers to an anti-tumor treatment that enhances or boosts the immune response against the tumor cells. Immunotherapies include cell-based immunotherapies, for example administration of immune cells that are able to recognize tumor cells, such as chimeric antigen receptor (CAR) T cells and NK cells, or T cells having a TCR specific for a tumor antigen, or antigen-presenting cells (APCs such as dendritic cells) capable of expressing tumor antigens at their surface. Immunotherapies also include the administration of specific antibodies that recognize antigens expressed by tumor cells and target them for destruction by the immune system, or the administration of cytokines (interferons, interleukins) that stimulates the immune response. Another type of immunotherapy comprises administration of an immune checkpoint inhibitor. A combination of different types of immunotherapies may be used, for example administration of immune cells (CAR T or NK cells) in combination with an immune checkpoint inhibitor.

The term "immune checkpoint inhibitor" (ICI) or "immune checkpoint blocker" (ICB) as used herein refers to an agent that block or inhibit the activity of a negative regulator of the immune response. Examples of such negative regulators of the immune response (i.e. immune checkpoint) include Adenosine A2A receptor (A2AR), B7-H3 (CD276), B7-H4 (VTCN1), B and T Lymphocyte Attenuator (BTLA or CD272), Cytotoxic T-Lymphocyte-Associated protein 4 (CTLA-4, CD152), Indoleamine 2,3-dioxygenase (IDO), Killer-cell Immunoglobulin-like Receptor (KIR), Lymphocyte Activation Gene-3 (LAG3), nicotinamide adenine dinucleotide phosphate NADPH oxidase isoform 2 (NOX2), Programmed Death 1 (PD-1) receptor, PD-L1. PD-L2, T-cell Immunoglobulin domain and Mucin domain 3 (TIM-3), V-domain Ig suppressor of T cell activation (VISTA), and Sialic acid-binding immunoglobulin-type lectin 7 (SIGLEC7 or CD328) and SIGLEC9 (CD329). In an aspect, the immune checkpoint inhibitor is an inhibitor of CTLA-4, PD-1 or PD-L1. In an aspect, the immune checkpoint inhibitor is an inhibitor of PD-1, such as an anti-PD-1 antibody. In an aspect, the immune checkpoint inhibitor is an inhibitor of PD-L1, such as an anti-PD-L1 antibody. In an aspect, the immune checkpoint inhibitor is an inhibitor of CTLA-4, such as an anti-CTLA-4 antibody.

The cancer may be any type of cancer, including a primary (or original) cancer, a relapsing cancer or a metastatic cancer. Examples of cancers include heart sarcoma, lung cancer, small cell lung cancer (SCLC), non-small cell lung cancer (NSCLC), bronchogenic carcinoma (squamous cell, undifferentiated small cell, undifferentiated large cell, adenocarcinoma), alveolar (bronchiolar) carcinoma, bronchial adenoma, sarcoma (*e.g*., Ewing's sarcoma, Karposi's sarcoma), lymphoma, chondromatous hamartoma, mesothelioma; cancer of the gastrointestinal system, for example, esophagus (squamous cell carcinoma, adenocarcinoma, leiomyosarcoma, lymphoma), stomach (carcinoma, lymphoma, leiomyosarcoma), gastric, pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); cancer of the genitourinary tract, for example, kidney cancer (adenocarcinoma, Wilm's tumor [nephroblastoma], lymphoma, leukemia), bladder and/or urethra cancer (squamous cell carcinoma, transitional cell carcinoma, adenocarcinoma), prostate cancer (adenocarcinoma, sarcoma), testis cancer (seminoma, teratoma, embryonal carcinoma, teratocarcinoma, choriocarcinoma, sarcoma, interstitial cell carcinoma, fibroma, fibroadenoma, adenomatoid tumors, lipoma); liver cancer, for example, hepatoma (hepatocellular carcinoma, HCC), cholangiocarcinoma, hepatoblastoma, angiosarcoma, hepatocellular adenoma, hemangioma, pancreatic endocrine tumors (such as pheochromocytoma, insulinoma, vasoactive intestinal peptide tumor, islet cell tumor and glucagonoma); bone cancer, for example, osteogenic sarcoma (osteosarcoma), fibrosarcoma, malignant fibrous histiocytoma, chondrosarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; cancer of the nervous system, for example, neoplasms of the central nervous system (CNS), primary CNS lymphoma, skull cancer (osteoma, hemangioma, granuloma, xanthoma, osteitis deformans), meninges (meningioma, meningiosarcoma, gliomatosis), brain cancer (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); cancer of the reproductive system, for example, gynecological cancer, uterine cancer (endometrial carcinoma), cervical cancer (cervical carcinoma, pre-tumor cervical dysplasia), ovarian cancer (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma), fallopian tube cancer (carcinoma); placenta cancer, penile cancer, prostate cancer, testicular cancer; cancer of the hematologic system, for example, blood cancer (acute myeloid leukemia (AML), chronic myeloid leukemia (CML), acute lymphoblastic leukemia (ALL), chronic lymphocytic leukemia (CLL), myeloproliferative diseases, multiple myeloma, myelodysplastic syndrome), Hodgkin's disease, non-Hodgkin's lymphoma [malignant lymphoma]; cancer of the oral cavity, for example, lip cancer, tongue cancer, gum cancer, palate cancer, oropharynx cancer, nasopharynx cancer, sinus cancer; skin cancer, for example, malignant melanoma, cutaneous melanoma, basal cell carcinoma, squamous cell carcinoma, Karposi's sarcoma, moles dysplastic nevi, lipoma, angioma, dermatofibroma, and keloids; adrenal gland cancer: neuroblastoma; and cancers of other tissues including connective and soft tissue, retroperitoneum and peritoneum, eye cancer, intraocular melanoma, and adnexa, breast cancer (*e.g*., ductal breast cancer), head or/and neck cancer (head and neck squamous cell carcinoma), anal cancer, thyroid cancer, parathyroid cancer; secondary and unspecified malignant neoplasm of lymph nodes, secondary malignant neoplasm of respiratory and digestive systems and secondary malignant neoplasm of other sites.

Immune checkpoint inhibitors have been approved or are currently being tested in phase III and IV clinical trials for several cancers including lung cancer (*e.g*., non-small cell lung cancer (NSCLC) and small cell lung cancer, squamous cell lung carcinoma), head and neck cancer (*e.g*., head and neck squamous cell carcinoma, renal cell carcinoma, gastric adenocarcinoma, nasopharyngeal neoplasms, urothelial carcinoma, colorectal cancer, mesothelioma (*e.g*., pleural mesothelioma), breast cancer (*e.g*., triple-negative breast cancer, TNBC), esophageal neoplasms, multiple myeloma, gastric and gastroesophageal junction cancer, gastric adenocarcinoma, melanoma, Merkel-cell carcinoma (MCC), lymphoma (*e.g*., Hodgkin and non-Hodgkin lymphoma, diffuse Large B-cell lymphoma), liver cancer (*e.g*., hepatocellular carcinoma), melanoma, ovarian cancer, fallopian tube cancer, peritoneal neoplasms, bladder cancer, transitional cell carcinoma, prostatic neoplasms and biliary tract neoplasms (see, *e.g.,* Darvin et al., Experimental & Molecular Medicine volume 50, Article number: 165 (2018)). Thus, in an aspect, the cancer is one of the above-noted cancer for which immune checkpoint inhibitors have been approved or are currently being tested in phase III and IV clinical trials.

Currently approved immune checkpoint inhibitors include the anti-CTLA-4 Ipilimumab (melanoma and lung cancer), the anti-PD-1 Nivolumab (melanoma, lung cancer, renal cell carcinoma, Hodgkin lymphoma, head and neck cancer, colon cancer, and liver cancer), Pembrolizumab (melanoma, lung cancer, head and neck cancer, Hodgkin lymphoma, renal cell carcinoma and stomach cancer), and Cemiplimab (squamous cell skin cancer, myeloma, and lung cancer), and the anti-PD-L1 atezolizumab (NSCLC, small cell lung cancer, TNBC), Avelumab (NSCLC, MCC) and Durvalumab (urothelial carcinoma, lung cancer). Thus, in an aspect, the cancer is one of the above-noted cancer for which immune checkpoint inhibitors have been approved. In a further aspect, the cancer is resistant to PD-1 inhibitor-based therapy (anti-PD-1 therapy), and is melanoma, lung cancer, renal cell carcinoma, Hodgkin lymphoma, head and neck cancer, colon cancer, liver cancer, stomach cancer, squamous cell skin cancer or myeloma.

In an aspect, the above-mentioned treatment comprises the use/administration of more than one (*i.e.* a combination of) active/therapeutic agents, castalagin in combination with an immune checkpoint inhibitor (i.e. combination therapy). The combination of agents may be administered or co-administered (*e.g*., consecutively, simultaneously, at different times) in any conventional dosage form. Co-administration in the context of the present disclosure refers to the administration of more than one therapeutic in the course of a coordinated treatment to achieve an improved clinical outcome. Such co-administration may also be coextensive, that is, occurring during overlapping periods of time. For example, a first agent may be administered to a patient before, concomitantly, before and after, or after a second active agent is administered. The agents may in an aspect be combined/formulated in a single composition and thus administered at the same time. Alternatively, they may me formulated in separate compositions, and thus administered separately (at the same time or at different times).

In an aspect, the doses of castalagin and/or of the immune checkpoint inhibitor that are used/administered in the uses, compositions, combination therapy of the disclosure is a suboptimal dose. "Suboptimal dose" as used herein refers to a dose of one of the compound(s) of the combination described herein (castalagin and/or of the immune checkpoint inhibitor), which, when used in the absence of the other compound of the combination, results in a biological effect of 50% or less, in an aspect of 40% or less, in a further aspect of 30% or less, in a further aspect of 20% or less, in a further aspect of 10% or less. As such, use of a combination of the compounds described herein, where one or more compounds in the combination is used at a suboptimal dose, may achieve increased efficacy/biological effect relative to using the compound(s) in the absence of the other(s), at a comparable suboptimal dose.

As used herein, a synergistic effect is achieved when the effect of the combined compounds is greater than the theoretical sum of the effect of each agent in the absence of the other. One potential advantage of combination therapy with a synergistic effect is that lower dosages (*e.g*., a suboptimal dose) of one or both of the drugs or therapies may be used in order to achieve high therapeutic activity with low toxicity. In an aspect, the combination therapy (castalagin and the immune checkpoint inhibitor) results in at least a 5% increase in the effect relative to the predicted theoretical additive effect of the agents. In a further aspect, the combination therapy results in at least a 10% increase in the effect relative to the predicted theoretical additive effect of the agents. In a further aspect, the combination therapy results in at least a 20% increase in the effect relative to the predicted theoretical additive effect of the agents. In a further aspect, the combination therapy results in at least a 30% increase in the effect relative to the predicted theoretical additive effect of the agents. In a further aspect, the combination therapy results in at least a 50% increase in the effect relative to the predicted theoretical additive effect of the agents. A further advantage of using the drugs in combination is that efficacy may be achieved in situations where either drug alone would not have an effect, for example for a cancer or tumor resistant to the immune checkpoint inhibitor. Resistance means that the administration of the immune checkpoint inhibitor alone does not lead to a significant therapeutic effect, *e.g*. a significant reduction in tumor volume or tumor cell number. Examples of cancers for which resistance to immune checkpoint inhibitors has been reported in patients and/or animal models include lung cancer (*e.g*., NSCLC), pancreatic cancer, prostate cancer, melanoma, ovarian cancer, urothelial cancer, renal cell carcinoma (see, *e.g.,* Fares et al., American Society of Clinical Oncology Educational Book 39, 147-164, 2019; Pandey et al., Cancer Drug Resist 2019; 2:178-188).

The castalagin and/or the immune checkpoint inhibitor may be administered/used in combination with one or more additional active agents or therapies (chemotherapy, radiotherapy, surgery, vaccines, immunotherapy, etc.) for the treatment the targeted disease/condition (cancer) or for the management of one or more symptoms of the targeted disease/condition (*e.g*., pain killers, anti-nausea agents, etc.). In an aspect, the castalagin and/or the immune checkpoint inhibitor is/are used in combination with one or more chemotherapeutic agents, immunotherapies (*e.g*., using CAR T cells or CAR NK cells), antibodies, cell-based therapies, etc. Examples of chemotherapeutic agents suitable for use in combination with the castalagin and/or the immune checkpoint inhibitor include, but are not limited to, vinca alkaloids, agents that disrupt microtubule formation (such as colchicines and its derivatives), anti-angiogenic agents, therapeutic antibodies, EGFR targeting agents, tyrosine kinase targeting agent (such as tyrosine kinase inhibitors), transitional metal complexes, proteasome inhibitors, antimetabolites (such as nucleoside analogs), alkylating agents, platinum-based agents, anthracycline antibiotics, topoisomerase inhibitors, macrolides, retinoids (such as all-trans retinoic acids or a derivatives thereof); geldanamycin or a derivative thereof (such as 17-AAG), and other cancer therapeutic agents recognized in the art. In some aspects, chemotherapeutic agents for use in combination with the castalagin and/or the immune checkpoint inhibitor comprise one or more of adriamycin, colchicine, cyclophosphamide, actinomycin, bleomycin, duanorubicin, doxorubicin, epirubicin, mitomycin, methotrexate, mitoxantrone, fluorouracil, carboplatin, carmustine (BCNU), methyl-CCNU, cisplatin, etoposide, interferons, camptothecin and derivatives thereof, phenesterine, taxanes and derivatives thereof (*e.g*., taxol, paclitaxel and derivatives thereof, taxotere and derivatives thereof, and the like), topetecan, vinblastine, vincristine, tamoxifen, piposulfan, nab-5404, nab-5800, nab-5801, Irinotecan, HKP, Ortataxel, gemcitabine, Oxaliplatin, Herceptin^{®}, vinorelbine, Doxil^{®}, capecitabine, Alimta^{®}, Avastin^{®}, Velcade^{®}, Tarceva^{®}, Neulasta^{®}, lapatinib, sorafenib, erlotinib, erbitux, derivatives thereof, and the like.

The subject may be any animal, and more particularly mammals such as a mouse, a rat, a dog and a human. In an aspect, the subject is a human.

### MODE(S) FOR CARRYING OUT THE INVENTION

The present disclosure is illustrated in further details by the following non-limiting examples.

### Materials and Methods

*Murine studies.* All animal studies were approved by the Institutional Animal Care Committee (CIPA) and carried out in compliance with the Canadian Council on Animal Care guidelines. Murine experiments were conducted using seven-week-old female C57BL/6 mice, obtained from Charles River, Canada. Germ-free female C57BL/6 mice were purchased from the International Microbiome Centre Germ-Free Facility (University of Calgary, Canada) and maintained at the CR-CHUM Germ-Free Facility.

*Cell culture, reagents and tumor cell lines.* MCA-205 fibrosarcoma cells and E0771 mammary adenocarcinoma cells, class I MHC H-2^{b} syngeneic cell lines for C57BL/6 mice, were used for this study. MCA-205 cells were cultured at 37°C in the presence of 5% CO₂ in Roswell Park Memorial Institute (RPMI) 1640 (Gibco-Invitrogen) containing 10% Fetal bovine serum (FBS) (Wisent), 2mM L-glutamine (Wisent), 100IU/ml penicillin/streptomycin (Wisent), 1mM sodium pyruvate (Wisent) and MEM non-essential amino acids (Gibco-Invitrogen). E0771 cells were cultured at 37°C in the presence of 5% CO₂ in Dulbecco's Modified Eagle's Medium (DMEM) (Gibco-Invitrogen) containing 10% FBS (Wisent), 2mM L-glutamine, 100IU/ml penicillin/streptomycin (Wisent), 1 mM sodium pyruvate (Wisent).

*Subcutaneous model* of *MCA-205 sarcoma and E0771 breast cancer.* Syngeneic C57BL/6 mice were implanted with 0.8×10⁶ MCA-205 or 0.5x10⁶ E0771 subcutaneously. When tumors reached 20 to 35 mm² in size, mice were treated four times (or two times for flow cytometry analysis, see section below) intraperitoneally (*i.p*.) every three day with anti-PD-1 monoclonal antibody (mAb) (250 µg/mouse; clone RMP1-14, BioXcell) or isotype control (clone 2A3, BioXcell). Upon treatment initiation, mice received a daily oral gavage with the following products: *Myrciaria dubia,* Camu-camu (CC) raw extract (Sunfood) (200 mg/kg per mouse), fractions from the extraction round 1 (P, INT, NP, Insol) (40.18 mg/kg per mouse), fractions from the extraction round 2 (P1, P2, P3 and P4) (equivalent to fraction P dose of 40.18 mg/kg per mouse), vescalagin (extracted from CC, see isolation process below) (0.85 mg/kg per mouse), ellagic acid (0.85 mg/kg per mouse) (Sigma-Aldrich), urolithin A (0.85 mg/kg per mouse) (Sigma-Aldrich), *Castalin* (0.5 mg/kg per mouse) (PhytoProof^{©}, Sigma-Aldrich) and castalagin at different concentration: 1/8, 1/6, 1/4, 1/2 of the standard concentration, at the standard concentration (0.85 mg/kg per mouse), 1.5-fold (1.25 mg/kg per mouse) and three-fold increase concentration (2.55 mg/kg per mouse) (PhytoProof^{©}, Sigma-Aldrich or isolated from food grade oak, see isolation process below). In the control group mice received daily oral gavage with water (100 µL) Tumor area was routinely monitored every three days by means of a caliper. In the depletion experiments, we used the anti-CD8 mAb (150 µg/mouse; clone 53-6.7, BioXcell) or isotype control (clone 2A3, BioXcell.

*Antibiotic treatments.* For the antibiotics (ATB) experiments, mice were treated with an ATB solution containing ampicillin (1 mg/ml), streptomycin (5 mg/ml), and colistin (1 mg/ml) (Sigma-Aldrich) added to the sterile drinking water of mice as previously described (Routy et al, Science, 2018 Jan 5;359(6371):91-97. Epub 2017 Nov 2). Antibiotic activity was confirmed by cultivating fecal pellets resuspended in Brain heart infusion (BHI) medium +15% glycerol at 0.1 g/ml on COS (*Columbia Agar* with 5% *Sheep Blood*) plates for 48 hours at 37°C in aerobic and anaerobic conditions weekly. For the fecal microbiota transplantation (FMT) experiments in SPF-reared mice, mice received 3 days of the same combination of ATB prior to FMT.

*Fecal microbiota transplantation (FMT) experiments.* FMT was performed as previously published Routy et al. by thawing fecal material from 5 different Non-Small cell lung cancer (NSCLC) patients amenable to immune checkpoint blockers (ICI after appropriate ethics approval at the Centre de recherche du Centre hospitalier de l'Université de Montréal (CRCHUM) in Montreal. Patient records were retrospectively analyzed to identify their response status. Two weeks after FMT, tumor cells were injected subcutaneously and mice were treated with anti-PD-1 mAb or isotype control +/- CC, castalagin or water as described above.

*Flow cytometry analysis.* Tumors and spleens were harvested at 9 days after the first injection of anti-PD-1 mAb into mice bearing MCA-205 tumors and at 11 days after the first injection of anti-PD-1 mAb into mice bearing E0771 tumors. Excised tumors were cut into small pieces and digested in RPMI medium containing Liberase^{™} at 25 µg/mL (Roche) and DNase I at 150 Ul/mL (Roche) for 30 minutes at 37°C and then crushed and filtered twice using 100 and 70 µm cell strainers (Fisher Scientific). Spleens were crushed in RPMI medium and subsequently filtered through a 100 µm cell strainer. Two million tumor cells or splenocytes were pre-incubated with purified anti-mouse CD16/CD32 (clone 93; eBioscience) for 30 minutes at 4°C before membrane staining with anti-mouse antibodies for CD3 (145-2C11), CD4 (GK1.5), CD8 (53-6.7), CD44 (IM7), CD45 (30-F11), CD45RB (C363-16A), CD62L (MEL-14), Foxp3 (FJK-16s), CXCR3 (CXCR3-173), CCR9 (CW-1.2), PD-1 (29F.1A12), PD-L1 (MIH5), ICOS (7E.17G9) (BD, BioLegend, R&D and eBioscience). For the intracellular staining, the Foxp3 staining kit (eBioscience) was used. Dead cells were excluded using the Live/Dead Fixable aqua dead cell stain kit (Life Technologies). The samples were acquired on BD Fortessa 16 colors cytometer (BD) and analysis were performed with FlowJo software (BD).

*Immunofluorescence staining.* Murine tumors preserved in optimal cutting temperature (OCT) compound were cut (5 µm-thick sections) and adhered to microscope slides and stored at -80C°. Upon the start of the experiment, slides were air dried and washed with cold acetone. To prevent non-specific binding to biotin, the Endogenous Avidin Biotin Blocking kit (ThermoFisher) was used. Additionally, tissues were incubated with 10% donkey serum in order to reduce background staining. Primary antibodies used are anti-CD4, anti-CD8, anti-Foxp3. Donkey antigoat and donkey anti-rat conjugated to AF-488 were used as secondary antibodies and slides were incubated with Cy3-Streptavidin to detect biotinylated antibodies. Nuclei were visualized by counterstaining with DAPI (ThermoFisher). Images were generated using the whole slide scanner Olympus BX61VS (20x 0.75NA objective with a resolution of 0.3225 mm). The images were analysed using Visiomorph software (Visiopharm).

*HPLC and LC-MS systems.* A 1260 Infinity LC system connected to a 6120 Quadrupole LC/MS mass spectrometer from Agilent Technologies was used for reverse-phase chromatography (C18) and mass spectrometry (MS), respectively. X-Select SCH and HSS columns (Waters) were used for HPLC. For chromatography, two-component solvent system of MilliQ^{™} water (solvent A) and acetonitrile (ACN) (solvent B) were used, each acidified with 0.1% formic acid (FA). Only negative ionization data are reported as this polarity was optimal for polyphenols in acidified solutions.

*Camu-camu (CC) extraction.* Polyphenols in CC were extracted according to the procedure of Fracassetti et al., (Food Chem 2013 15;139(1-4):578-88) with slight modifications. Freeze-dried CC raw extract (SunFood) was extracted with 50% aqueous methanol (MeOH) in a 1:15 (g:mL) ratio (analytical experiments) or a 1:8 ratio (preparative experiments). The suspension was vortexed, sonicated, and incubated at room temperature for 60 min. The suspension was centrifuged, and supernatants were recovered. Second extraction were performed on the subsidence using 90% aqueous MeOH. The supernatants from both extracts were combined and filtered prior to analysis.

*Camu-camu (CC) extract analysis and LC-MS peak identification.* Following extraction of polyphenols from CC, the combined extract was injected into the LC-MS system for analysis. The solvent gradient used to resolve components in the sample was adapted from Fracassetti et al., 2013. The relative retention times at 254 nm and negative ion mass spectra from the LC-MS analysis were compared to those from the characterization of CC polyphenols by Fracassetti et al., 2013. The identities of peaks were tentatively assigned based on the agreement between the present data and the data reported by Fracassetti et al., 2013.

*Identification of the active fraction P isolated from camu-camu (CC), fractionation round* 1. To assess which components of CC were responsible for its activity, four fractions were produced by reversed-phase chromatography and sequential extraction: polar (P), intermediate polarity (M), non-polar (NP), and insoluble (INS). Polyphenols were extracted from CC and concentrated to dryness, then re-dissolved in a mixture of 40% ACN:10% MeOH in water to solubilize most polyphenols. Insoluble material was separated by filtration and discarded. The same solvent gradient used for CC peak identification (above) was used for preparative HPLC. Fractions were manually collected every 10 min for a total of 60 min. Fractions were then frozen at -80 °C and lyophilized. The three fractions from 30-60 min were combined to produce fraction NP. The HPLC column breakthrough from 0-10 min was used as a starting point to produce fraction P. Briefly, four Strata C18-E solid-phase extraction (SPE) columns (Phenomenex), were set-up in parallel and conditioned with MeOH. The lyophilized 0-10 min HPLC breakthrough was dissolved in MilliQ water to a concentration of 10 mg/mL. Five mL of sample (10 mg/mL) were added to each column and the flow-through was collected. Then, 9 mL of 5% ACN were added to the column and the flow-through was collected. Collected flow-throughs from each column were combined and lyophilized to produce fraction P. Fraction M and INS were produced through successive extractions of CC raw extract in water, to remove highly polar compounds, 50% MeOH, and 90% MeOH. Fraction M was composed of the 50% MeOH extract, which was evaporated, then lyophilized. The INS fraction was composed of the dried subsidence after all extraction steps were completed.

*Identification of the active fraction P3, fractionation round 2.* To assess which components of fraction P were responsible for its activity, 4 fractions were produced (P1, P2, P3 and P4). A new solvent gradient was developed to focus on polar polyphenols that were contained in fraction P. The gradient method was as follows: 0% B at 0 min, 16% B at 30 min, 95% B at 35 min, 100% B from 36-46 min. As for the production of fraction P, the HPLC breakthrough from 0-10 min (fractionation round 1), dissolved in MilliQ water was used as a starting point for fractionation round 2. Fractions were manually collected every minute for 30 min. Fractions for each run were analyzed by LC-MS, then lyophilized and combined to produce fractions P1, P2, P3 and P4 as follows: min 0-5 were combined to make fraction P1, min 5-17 were combined to make fraction P2, min 18-19 were combined to make fraction P3, and min 20-30 were combined to make fraction P4.

*Fraction P3 characterization.* The purity of the castalagin peak in fraction P3 was determined by peak integration of the 254 nm analytical LC-MS chromatogram. A castalagin analytical standard, dissolved in MilliQ water, was used for comparison of retention times at 254 nm and negative ion mass spectra. Both fraction P3 and the castalagin analytical standard were dissolved in D₂O for analysis by NMR. ¹H, ¹H-¹H correlated spectroscopy (COSY), and ¹H-¹³C heteronuclear single quantum coherence (HSQC) NMR spectra were recorded on a Bruker AVIIIHD 500 MHz NMR spectrometer. Peaks were compared to the castalagin structural reassignment by Matsuo et al., 2015 (Org Lett 2015 Jan 2;17(1):46-9. Epub 2014 Dec 12).

*Castalagin and vescalagin isolation from Camu-camu (CC) and from the food grade oak.* Twenty grams of freeze-dried CC powder were extracted as described above. The crude extract was subjected to pre-fractionation using Strata C18-E SPE columns. Briefly, 3 mL of re-dissolved crude extract were loaded onto an SPE column and 2 mL of MilliQ water were added to remove ascorbic acid. Then, 9 mL of 5% ACN were added to each column and the flow-through was collected in 3 mL batches. Castalagin and vescalagin were subsequently purified by HPLC from the flow-throughs. Isolates were analyzed by LC-MS and their purity was assessed.

*Fluo-castalagin synthesis, purification, and characterization.* Castalagin was mono-functionalized with fluorescein *via* a transesterification reaction with 5/6-carboxyfluorescein succinimidyl ester (Fluorescein-NHS). Briefly, castalagin was dissolved in DMF, then reacted with Fluorescein-NHS (2 eq.) in the presence of triethylamine (2 eq.), and 4-dimethyaminopyridine. Following workup with DOWEX 50WX8 resin, the crude mixture was analyzed by LC-MS. The peaks corresponding to mono-functionalized Fluo-castalagin were isolated by HPLC. Bacteria R. *bromii, E. coli and B. thetaoitomicron* were staining in the presence of fluorescein bound castalagin at 37°C and 0°C and in the presence of unbound castalagin at 100 x concentration.

*Inversed epifluorescence microscopy.* An inverted optical microscope (Ti2, Nikon, Inc.) configured for epifluorescence and equipped with a high-sensitivity CCD camera (C14440-20UP, Hamamatsu, Inc.) was used to acquire images of the fluo-castalagin staining.

*Genomic DNA extraction from mouse feces.* Total genomic DNA from fecal pellets was extracted using ZymoBIOMICS DNA Miniprep Kit (Zymo Research Corporation) and immediately stored at -80°C. This protocol involves a bead beating step to ensure full recovery of bacterial DNA. DNA concentration and quality were measured using the Nanodrop ND-1000 (ThermoFisher).

*Quantitative real-time PCR (qRT-PCR).* Quantitative real-time PCR was performed to evaluate the relative levels of the total bacterial DNA and the V6 region of the 16S rRNA gene was amplified using the primer set 891F (5'-TGGAGCATGTGGTTTAATTCGA-3', SEQ ID NO:1) and 1033R (5'-TGCGGGACTTAACCCAACA-3', SEQ ID NO:2) (Anhê et al. Diabetologia. 2018 Apr;61(4):919-931) and specifically to evaluate the relative levels of the Ruminococcaceae DNA using the specific primers F (5'- ACTGAGAGGTTGAACGGCCA-3', SEQ ID NO:3) and R (5'-CCTTTACACCCAGTAAWTCCGGA-3', SEQ ID NO:4) (Garcia-Mazcorro JF et al FEMS Microbiol Ecol 2012;80(3):624-36). DNA (400ng/well) extracted was combined with 500 nM of the above described primer mix and 1X qPCRBIO SyGreen blue Mix Hi-ROX (PCRBIOSystems). The qPCR reaction was performed on the Applied Biosystems StepOnePlus Real-Time PCR System (ThermoFisher Scientific) at 95°C for 3min to denature DNA, with amplification proceeding for 40 cycles at 95°C for 5s, 60°C for 30 s, and completed with a melt curve stage. Raw threshold cycle (Ct) values were compared to a bacterial standard curve produced with Escherichia coli DNA for the 16s analysis and with *Ruminoccocus bicirculans* for the *Rumniccocaceae* analysis for approximation of bacterial load.

*16S rRNA gene sequence processing and analysis mouse feces samples.* Isolated DNA was analyzed using 16S ribosomal RNA (rRNA) gene sequence to investigate the microbial composition in fecal samples. The V3-V4 region of the 16S rDNA gene was amplified by PCR using primers Bakt_341F (5'-CCTACGGGNGGCWGCAG-3', SEQ ID NO:5) and Bakt_805R (5'-GACTACHVGGGTATCTAATCC-3', SEQ ID NO:6) adapted to incorporate the transposon-based Illumina Nextera adapters (Illumina) and a sample barcode sequence allowing multiplexed paired-end sequencing. PCR mixtures contained 1× Q5 buffer (NEB), 1× Q5 Enhancer (NEB), 200 µM dNTP (VWR International), 0.2 µM of forward and reverse primer (Integrated DNA Technologies), 1 unit of Q5 (NEB) and 1 µl of template DNA in a 50 µl reaction. The PCR cycling conditions consisted of an initial denaturation of 30 sec at 98°C, followed by a first set of 15 cycles (98°C for 10 sec, 55°C for 30 sec and 72°C for 30 sec), then by a second step of 15 cycles (98°C for 10 sec, 65°C for 30 sec and 72°C for 30 sec) and final elongation of 2 min at 72°C before cooling to 4°C indefinitely. PCR products were purified using 35 µl of magnetic beads (AxyPrep Mag PCR Clean up kit; Axygen Biosciences) per 50 µl PCR reaction. Amplifications were controlled on a Bioanalyzer 2100 using DNA 7500 chips (Agilent Technologies). Samples were pooled at an equimolar ratio; the pool was repurified as described before and checked for quality on a Bioanalyzer 2100 using a DNA high sensitivity chip. The pool was quantified using picogreen (Life Technologies) and loaded on a MiSeq system (Illumina). High-throughput sequencing was performed at the IBIS (Institut de Biologie Intégrative et des Systèmes - Université Laval).

Gene sequence processing and analysis was performed using R v4.0.0. DADA2R package v1.16.0 (Callahan et al., Nat Methods. 2016 Jul; 13(7):581-3) was used to generate exact amplicon sequence variants (ASV) of each sample from raw amplicon sequences. Sequences were corrected for Illumina amplicon sequence errors, de-replicated, chimera removed, and merged of paired-end reads with 260-bases for forward reads and 190-bases for reverse reads. The taxonomy assignment was performed against the SILVA reference database v138 (Quast et al., Nucleic Acids Res. 2013 Jan;41(Database issue):D590-6). Archea and Eukaryota residual sequences were removed. Downstream analyses were performed at the genus-level through phyloseq R package v1.32.0 (McMurdie et al., PLoS One. 2013 Apr 22;8(4):e61217). The alpha-diversity was estimated with the Shannon diversity index and the Inverse Simpson index. These indexes were compared between groups using Mann-Whitney tests.

*Statistical analysis.* Statistical analysis was performed using R v4.0.0. The Mann-Whitney U test was used to determine significant differences among the different groups using alpha-diversity which show the diversity in each individual sample measures. DESeq2 (Love et al., Genome Biol. 2014;15(12):550) was used to perform differential abundances analysis at the genus-level. Spearman rank correlation test was obtained using Graphpad Prism 8 was used to compare continuous variables between the flow cytometry analysis parameters and to the significant bacteria identified with differential abundance analysis in the water vs CC, water/αPD-1 vs CC/αPD-1, water/αPD-1 vs Castalagin/αPD-1, water/IsoPD-1 vs. Castalagin/αPD-1 groups for the MCA-205 and E0771 tumor model. Unless stated, all p-values are reported after Bonferroni correction when the question is addressing more than 2 experimental conditions. *p*-values were two-sided with 95% confidence intervals **p* < 0.05, ***p* < 0.01, ****p* < 0.001.

### Example 1: Administration of a Camu camu (CC) extract potentiates or restore the anti-PD-1 antitumor activity in murine tumor models

Aiming to find approaches to potentiates or restore the antitumor activity of ICB such as anti-PD-1 antibodies, a crude extract from Camu camu (*Myrciaria dubia*), an Amazonian fruit with a unique phytochemical profile, was administered in combination with an anti-PD-1 in syngeneic C57BL/6 mice implanted with MCA-205 sarcoma tumor cells (anti-PD-1 sensitive) according to the protocol depicted in **FIG. 1A****.** The Camu camu extract used in the experiments described herein is the Camu camu raw Powder commercialized by Sunfood, which is obtained from Camu camu berries from the South American rainforest that are dried and milled into a fine powder at a low temperature.

The results depicted in **FIGs. 1B-1C** show that daily oral gavage with the CC extract alone exhibits an anti-cancer activity (similar to anti-PD-1 monotherapy), and potentiates the anticancer activity of the anti-PD-1 antibody, as evidenced by a reduction in tumor size.

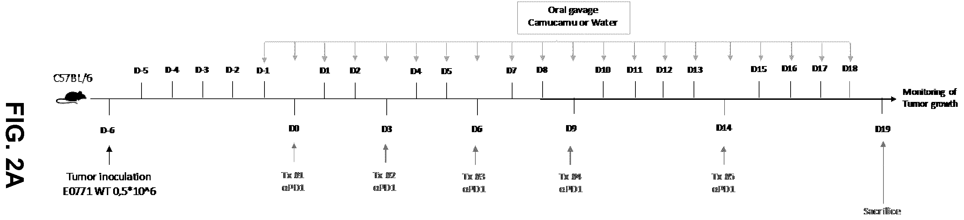

The antitumor effect of CC was next tested in mice implanted with an anti-PD-1-resistant tumor (E0771 mammary carcinoma) according to the protocol depicted in **FIG. 2A****.** As expected, administration of the anti-PD-1 alone did not lead to a significant reduction in tumor size in this model, confirming the resistance of E0771 mammary carcinoma cells to anti-PD-1 monotherapy **(****FIGs. 2B-2C****).** Similarly, administration of the CC extract alone failed to significantly reduce E0771 tumor size. However, a significant reduction of E0771 tumor size was obtained following administration of both the anti-PD-1 and CC extract **(****FIGs. 2B-C),** providing evidence that the CC extract has the ability to restore the anti-PD-1 antitumor response against anti-PD-1-resistant tumors.

### Example 2: The Camu-camu extract acts through modulation of the gut microbiota

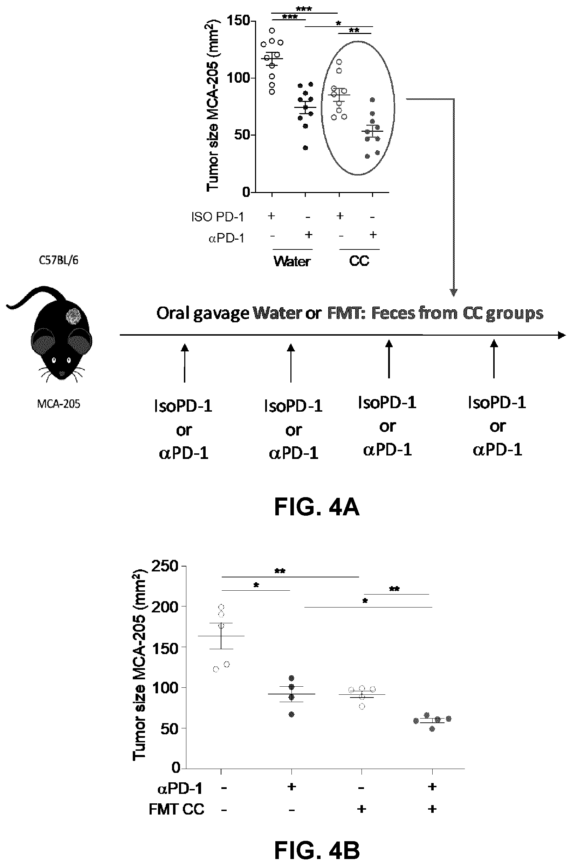

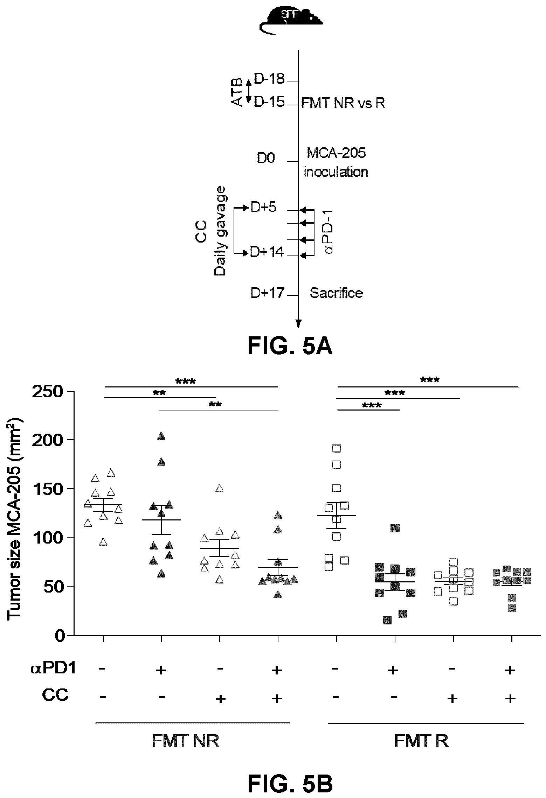

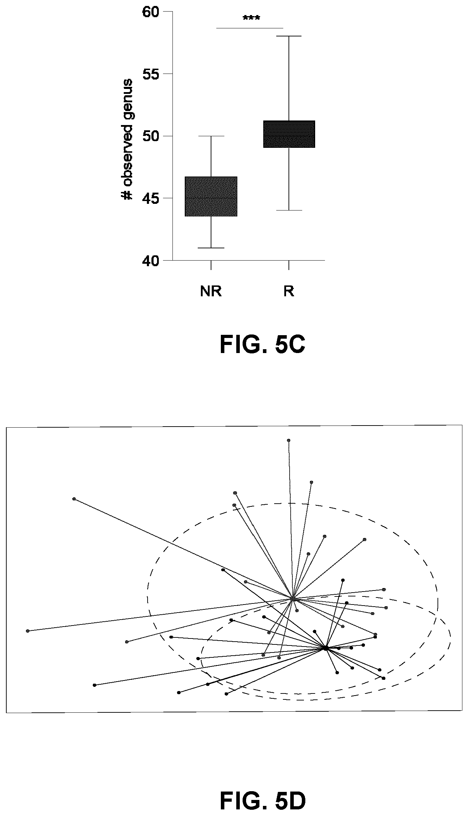

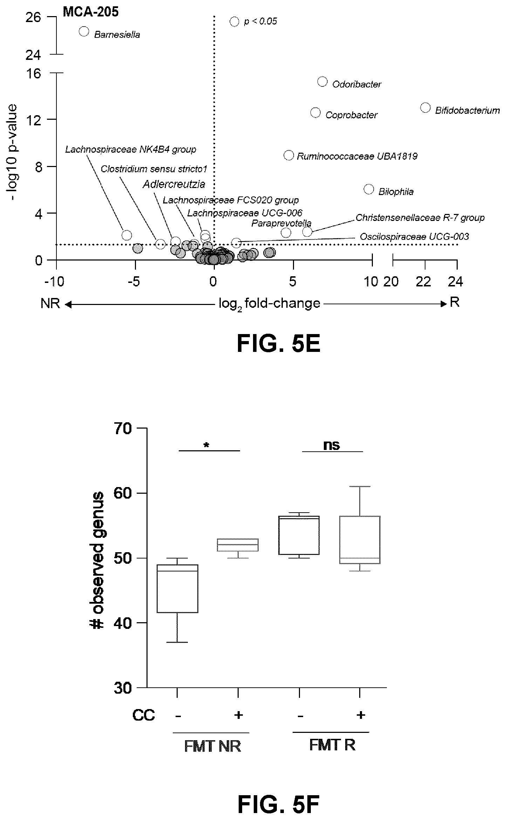

Experiments were performed according to the protocol depicted in **FIG. 3A** in order to better understand the mechanism by which the CC extract exerts its antitumor effect. First, the results depicted in **FIGs. 3B-C** show that administration of broad spectrum antibiotics (ATB), which affect the gut microbiota, completely abrogates the antitumor effect of the CC extract in the murine MCA-205 tumor model. Secondly, fecal microbiota transfer (FMT) experiments were performed in specific-pathogen-free (SPF) mice. More specifically, feces from mice previously treated with the CC extract were transferred into mice implanted with MCA-205 tumors, and the effect on tumor size was measured **(****FIG. 4A****).** As shown in **FIG. 4B****,** the transfer of microbiome from mice previously treated with the CC extract was sufficient to restore CC activity in monotherapy or combined with anti-PD-1. To explore the therapeutic potential of CC, ATB-treated mice were recolonized by performing FMT from two responder (R) patients and two nonresponder (NR) patients with non-small cell lung carcinoma (NSCLC). MCA-205 tumors were inoculated in these 'avatar' mouse models, and mice were treated with CC or water with or without αPD-1 **(****FIG. 5A****).** FMT from NR patients conferred resistance to αPD-1, whereas FMT from R patients restored the αPD-1 antitumor effect **(****FIGs. 5A** and **5B****).** Upon FMT engraftment (prior to CC +/- αPD-1), the baseline microbiome in mice that received FMT was characterized. FMT from R was associated with greater *alpha*-diversity **(****FIG. 5C****).** Two objective clusters were also found when analyzing beta-diversity R compared to NR groups **(****FIG. 5D****).** Interestingly, *Bilophilia* and *Ruminococcaceae UBA1819* were overrepresented in mice that underwent FMT from R and had tumors that were sensitive to water/αPD-1 **(****FIG. 5E****).**

In the FMT NR avatar mice, oral supplementation of CC/isoPD-1 reinstated the antitumor effect of CC **(****FIG. 4B****).** Furthermore, the combination of CC/αPD-1 restored αPD1 efficacy that was impaired in FMT NR mice treated with water/αPD-1. Conversely, in FMT R avatar mice, CC alone or in combination with anti-PD-1 did not exhibit any further enhancement of the antitumor response compared to water/αPD-1. At the microbiome level, CC/isoPD-1 increased the *alpha-*diversity in the FMT NR avatar, but did not affect the diversity in FMT R avatar mice in water/isoPD-1 **(****FIG. 5F****).** At the genus level, addition of CC/isoPD-1 to FMT NR was associated with increase in *Ruminococcaceae* relative abundance (p=0.055).

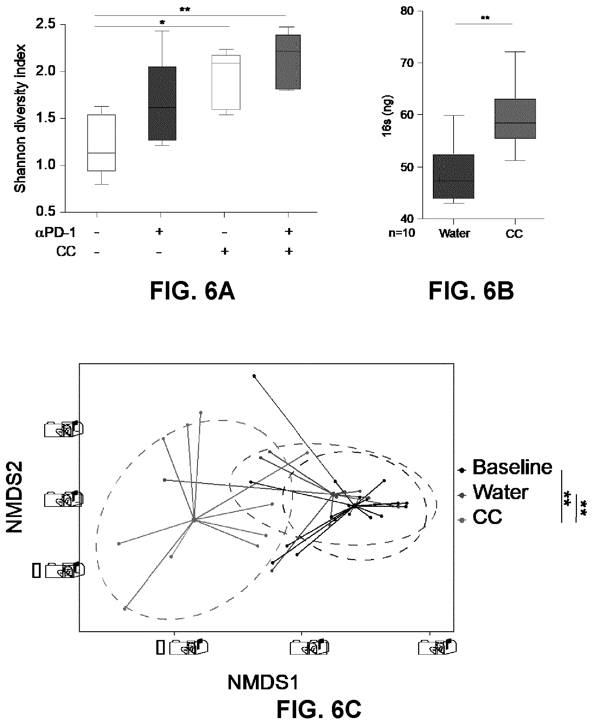

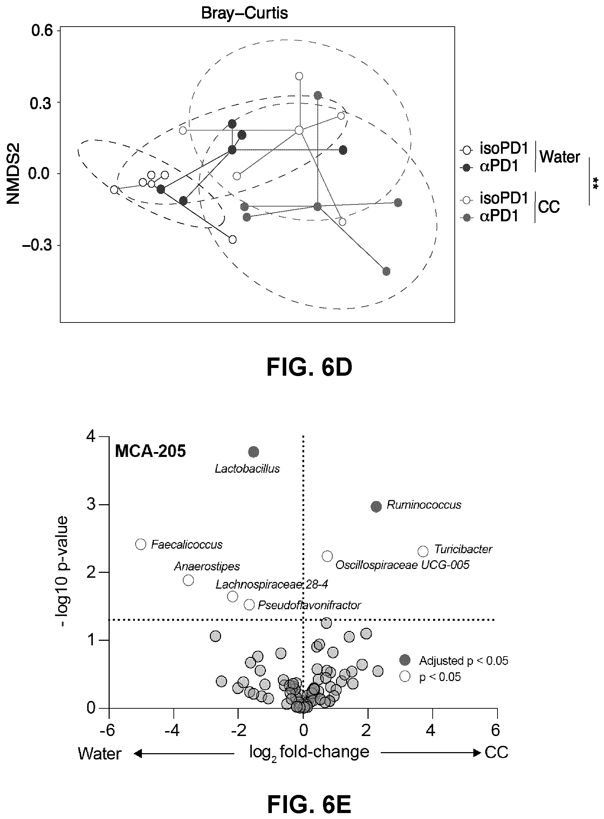

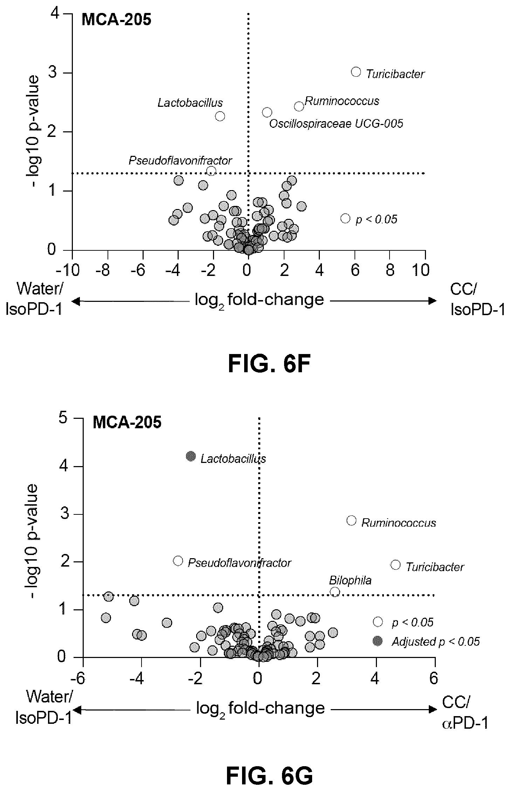

Next, microbiome profiling with 16s rRNA sequencing was performed on fecal sample from experiment described in **FIG. 1A****.** The V3-V4 region of the 16S rDNA gene was amplified by PCR using the primers Bakt_341F and Bakt_805R that have been adapted to incorporate the transposon-based Illumina Nextera adapters (Illumina). High-throughput sequencing was performed at the Institut de biologie intégrative et des systèmes (IBIS). After dataset filtration (lowhigh read), rRNA sequences that successfully pass the pre-processing steps and present with ≥97% nucleotide sequence identity were binned into Operational Taxonomic Units (OTU) using USEARCH 61 (version 6.1.544). These experiments revealed that CC treatment was associated with increased alpha diversity compared to water/isoPD-1, independent of αPD-1 therapy **(****FIG. 6A****).** Quantitative real-time (qRT-PCR) PCR with 16S rRNA gene-based specific primers confirmed an increased in bacterial abundance in the CC group compared to water **(****FIG. 6B****).**

*Beta*-diversity measured by Bray Curtis revealed that oral gavage with CC led to the development of separate bacterial clusters compared to pre-supplementation of CC (*p <* 0.001) **(****FIG. 6C****),** whereas oral gavage with water/αPD-1 showed no division of the microbiome into different clusters (p=0.16) **(****FIGs. 6C-D).**

Differential abundance analysis showed that specific bacteria at the genus level were specifically enriched in the CC groups compared with the water groups. *Ruminococcus* (adjusted p<0.05) was the most differentially abundant bacteria, followed by *Turicibacter* and *Oscillospiraceae UCG* 005 (non-adjusted p<0.05) **(****FIG. 6E****).** Moreover, *Ruminococcus* was the only bacteria that was consistently increased in both CC/isoPD1 as well as CC/αPD-1 groups compared to respective water groups **(****FIGs. 6F-G).**

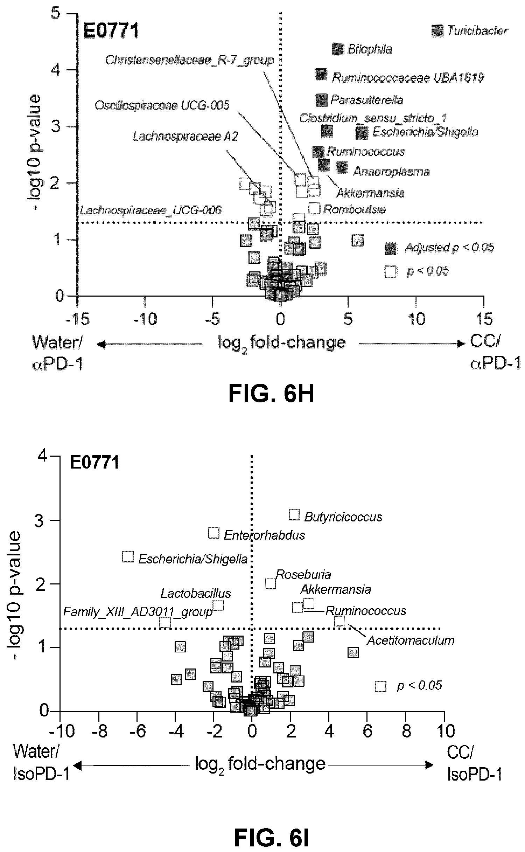

Profiling of the gut microbiome using 16s rRNA sequencing in the αPD-1-resistant E0771 tumor model showed that *Turicibacter, Bilophila, Ruminococcaceae UBA1819*, *Parasutterella, Clostridium sensu stricto 1, Ruminococcus, Akkermansia, Anaeroplasma* (adjusted p<0.05) were overrepresented in mice treated with CC/αPD-1 compared to water/αPD-1 **(****FIG. 6H****).** Interestingly, in mice treated with CC/isoPD-1 *Akkermansia* and *Ruminococcus* were also overrepresented compared to water/isoPD-1 **(****FIG. 6I****).** Altogether, these results revealed a specific association between bacteria species and the anticancer effect of CC.

These results provide compelling evidence that the CC extract's antitumor activity is dependent, at least in part, on the gut microbiota.

### Example 3: Effect of CC administration on immune cells

Immune surrogate profiling was performed in the murine tumor models to assess the effect of the treatment on immune cells. Administration of the CC extract alone or in combination with anti-PD-1 led to a significant upregulation of central memory (T_{CM}) CD8⁺ **(****FIGs. 7A****),** as well as a significant increase in the ratio of CD8⁺/Foxp3⁺CD4⁺ T (Treg) in three groups with antitumor efficacy namely CC/isoPD-1, CC/αPD-1 or water/αPD-1 was increased relative to water/isoPD-1 **(****FIG. 7B****)** in the MCA-205 tumor model. Furthermore, a significant increase of ICOS expression on CD8⁺ T cells was also observed in the E0771 tumor model administered with the CC extract alone or in combination with anti-PD-1 **(****FIG. 7C****).**

To verify that the antitumor activity associated with CC was mediated by CD8⁺ T cells, MCA-205 bearing mice received CC and anti-CD8⁺ monoclonal antibodies to deplete the CD8⁺ subpopulation and showed increased tumor growth compared to CC/isoCD8 (control), indicating that the antitumor effect of CC was CD8⁺ T cell-dependent **(****FIG. 7D****).**

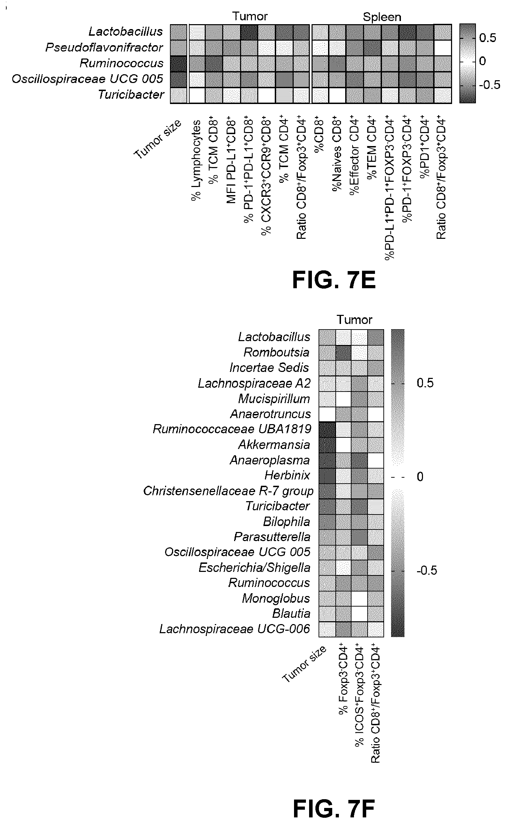

The impact of CC on the gut microbiome, tumor size and immune profiling in both tumor and spleen in the MCA-205 murine model was next examined. Pairwise comparisons using nonparametric spearman correlation between bacteria enriched in CC/isoPD-1 *versus* water/isoPD-1 group were performed with intratumoral cytometry immune markers and tumor size. Increase in the proportion of CD3⁺T cell infiltrate, CD8⁺T cell PD-L1 expression, CD8⁺ TCM cells and ratio of CD8⁺/Tregs was associated with bacteria enriched by CC, such as *Ruminoccocus* and downregulated *Lactobacillus* and *Pseudoflavonifractor* in MCA-205 **(****FIG. 7E****).** Similarly, in the CC/isoPD-1 group, CD8⁺ cells and ratio CD8⁺/Tregs in the splenocytes correlated with *Ruminoccocus* and *Oscillospiraceae* UCG 005 relative to water/isoPD-1.

In parallel, the TILs in the E0771 tumor were analyzed post-CC +/- αPD-1. CC combined with αPD-1 induced activation of intratumoral CD8⁺ T cells, as evidenced by the increase MFI of ICOS⁺CD8⁺T cells when compared to water/αPD-1 **(****FIG. 7C****).** Spearman rank correlations where then performed between bacteria upregulated in water/αPD-1 and CC/αPD-1, immune marker and tumor size which further demonstrated a positive correlation between the CD8/Treg ratio, and upregulation of ICOS⁺Foxp3⁻CD4⁺ T cells infiltration with the abundance of *Rumincoccus, Bilophila* and *Akkermansia* conferring a reduced tumor size **(****FIG. 7F****).**

### Example 4: Isolation of castalagin polyphenol extract as the bioactive compound of the anti-tumor activity of CC

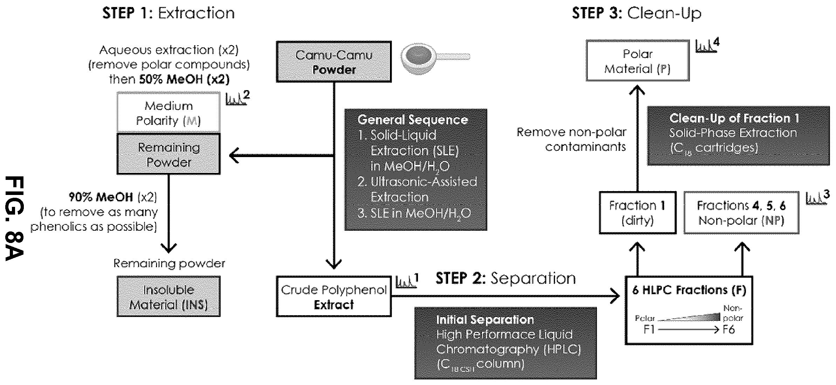

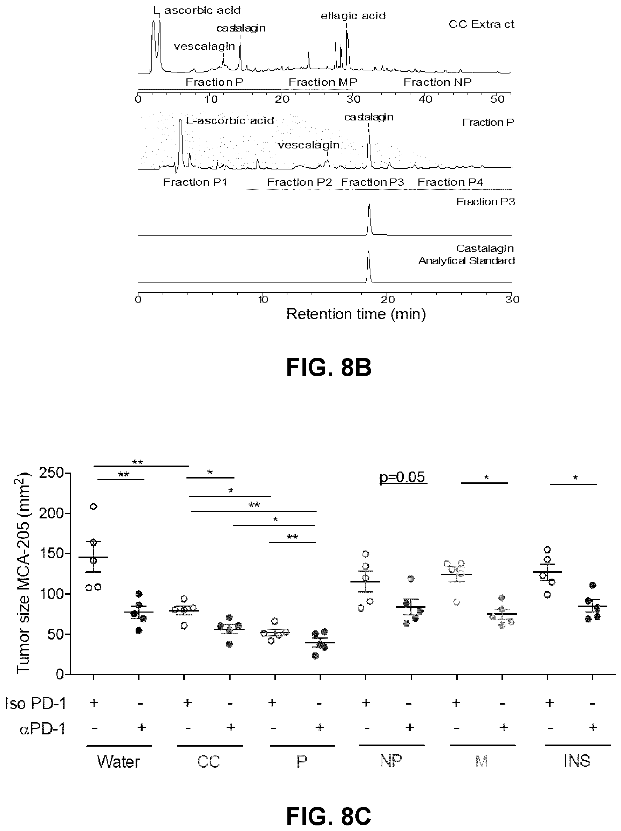

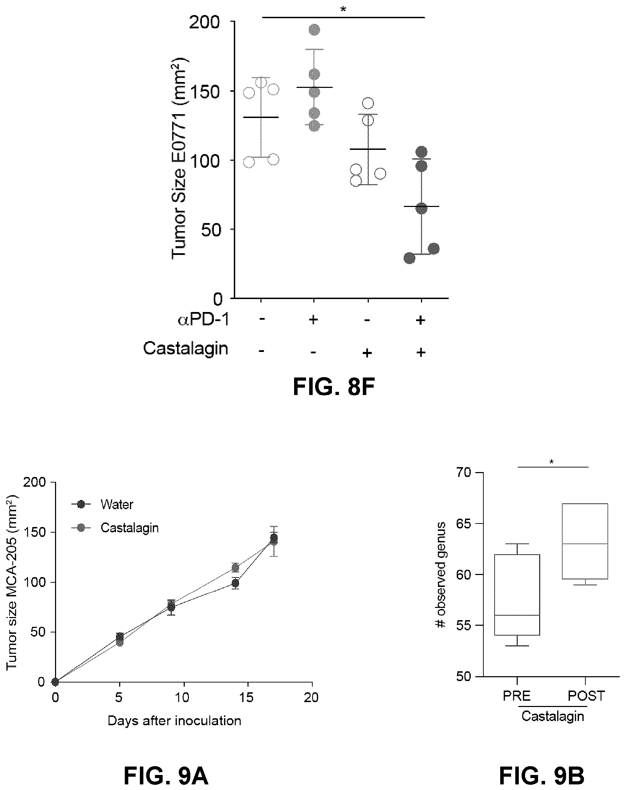

To identity the specific compound(s) in the CC extract conferring the antitumor activity observed in the murine tumor models, HPLC separation of the CC extract was performed according to the fractionation workflow diagram depicted at **FIG. 8A****.** A representative diagram of the HPLC retention time of the complete Camu-camu extraction, followed by HPLD retention time in the polar fraction and fraction P3 as well as castalagin extracted from oak is depicted in **FIG. 8B****.** Using this technique, the CC extract was first separated in 4 fractions (P-Polar, M-Intermediate/medium polar, NP-non polar, INS- insoluble) and tested each fraction in the MCA-205 tumor model. The results depicted in **FIG. 8C** showed that only the polar fraction (P) was able to mimic the effect of the CC extract tested in parallel in this experiment at a dose of 200 mg/kg.

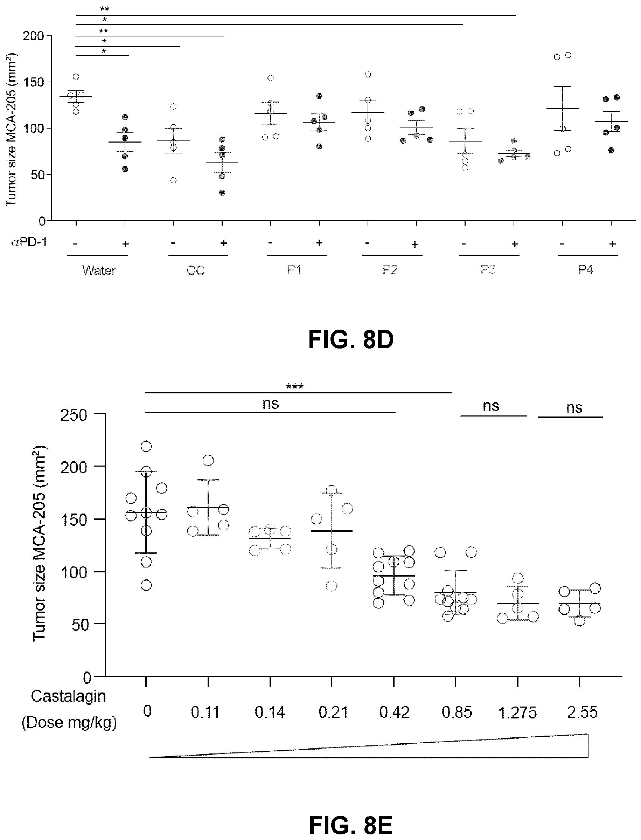

The active polar fraction P was then further separated in different sub-fractions according to retention time (P1-P4), which were subjected to HPLC analysis. The P1 fraction was mostly composed of ascorbic acid, P2 was composed of the polyphenol vescalagin and galic acid, P3 was composed of polyphenol castalagin, and P4 was composed of different impurities. These four sub-fractions were tested in the MCA-205 model and it was found that P3 mainly composed of castalagin was the only fraction that was associated with an antitumor effect similar to CC **(****FIG. 8D****).**

To confirm that the active ingredient in subfraction D was indeed castalagin, additional tests were performed using purified castalagin obtained from a commercial source (Phytoproof^{®} Reference substance from Millipore Sigma) as well as castalagin extracted from CC and oak using HPLC. HPLC on the various sources of castalagin confirmed that the retention time was similar to the P3 fraction extracted from CC. Using castalagin from PhytoProof^{®} or from oak at similar concentrations present in CC (0.85 mg/kg per mouse), a similar tumor inhibition was obtained in MCA-205 as well as in E0711 in combination with αPD-1 **(****FIGs. 8E-F).** To define if castalagin efficacy was dose-dependent, castalagin oral gavage was performed in mice using 6 different concentrations from 1/8 to 3-fold the standard dose. Some anti-cancer activity was observed at the 1/2 dose (0.42 mg/kg), and the anti-cancer activity became significant only at the standard dose (0.85 mg/kg, **FIG. 8E****).** On the other hand, a 3-fold increase concentration (2.55 mg/kg per mouse) did not the standard dose. Altogether, the results showed that castalagin is the bioactive compound of CC and had a dose-dependent effect with a potential plateau.

### Example 5: Castalagin supplementation increases bacterial diversity of the gut microbiome and enhances T cell-mediated ICI response

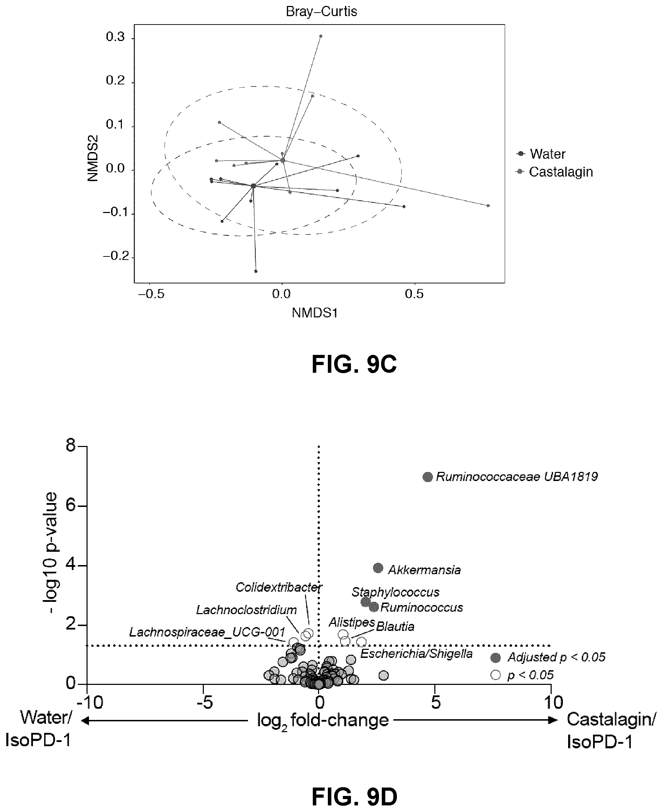

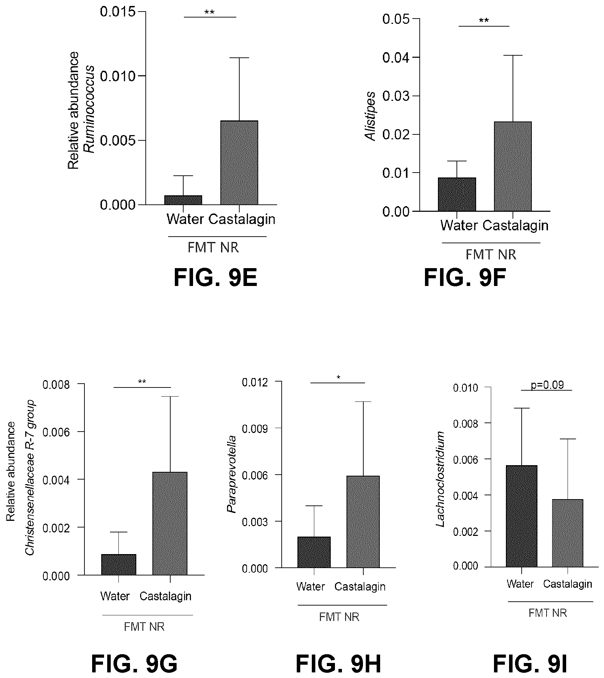

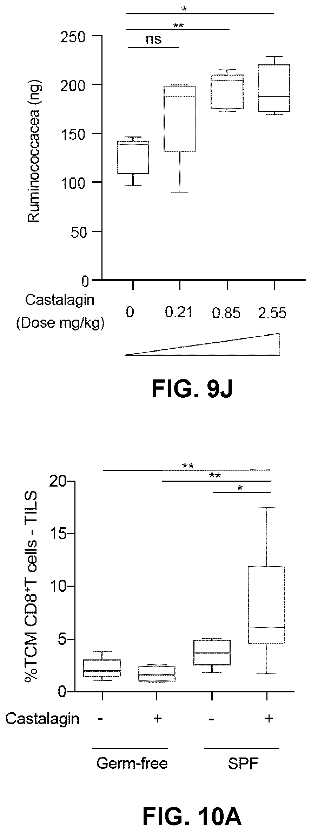

A proof-of-principle experiment to define the microbiome-dependent effect of castalagin in germ-free conditions was performed. As shown in **FIG. 9A****,** performing the experiment in germfree conditions abrogated castalagin antitumor effect. The impact of the microbiome of castalagin in SPF mice treated with castalagin/isoPD-1 was next assessed. 16S microbiome profiling revealed increased *alpha*-diversity **(****FIG. 9B****)** post-castalagin as well as significant clusters formation observed with beta-diversity **(****FIG. 9C****).** At the taxa level, castalagin led to the enrichment of *Akkermensia, Ruminococcaceae UBA1819, Ruminococcus, Staphylococcus, Escherichia*/*shigella, Blautia* and *Alistipes* while feces from water group were enriched with *Lachnospiraceae UCG-001* **(****FIG. 9D****).** Furthermore, an increase of the relative abundance after 16s sequencing analysis of *Ruminococcus, Alistepes, Christensenellaceae R7 group, Paraprevotella* in castalagin groups in the NR FMT experiment relative to water groups were observed while no difference of Lachnoclostridium was observed. between water and castalagin groups in the NR FMT experiment **(****FIG. 9E-I).** qRT-PCR with *Ruminococcaceae*-specific primers was performed on the feces on the dose-dependent castalagin experiment **(****FIG. 9J****).** *Ruminococcaceae* were not increase in the 1/4 concentration dose of castalagin (which has no antitumor effect) relative to the water control, while *Ruminococcaceae* abundance was significantly increased following supplementation of the standard castalagin dose or with three-fold standard castalagin dose (with antitumor effect) **(****FIG. 9J****).**

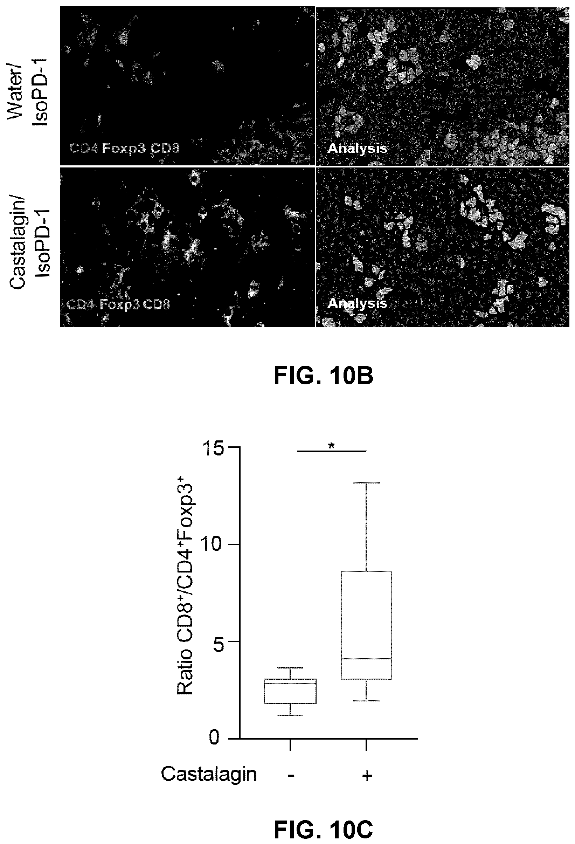

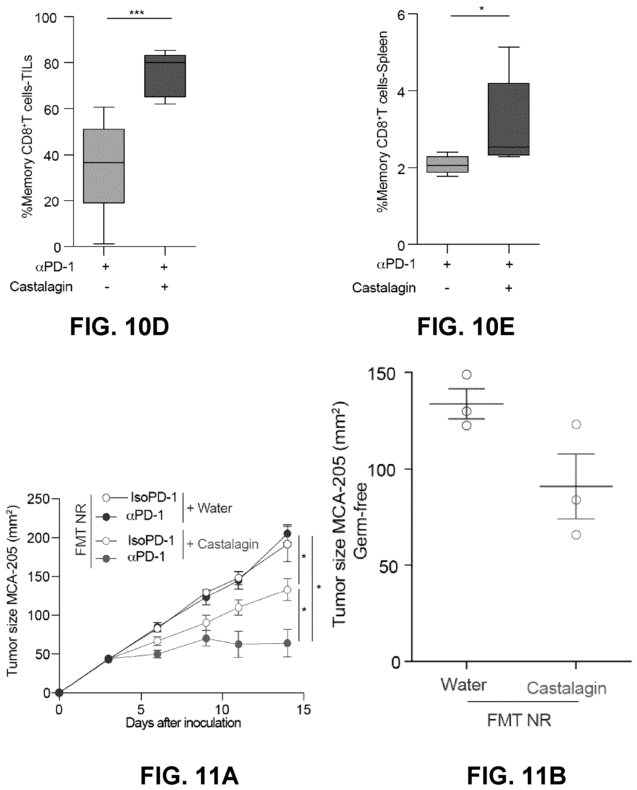

To determine if castalagin had the same impact on the systemic immune response than CC, two techniques were used. First, flow cytometry analysis in MCA-205 experiments revealed again an upregulation of T_{CM} CD8⁺ T cells while no impact of CC was observed in germfree condition **(****FIG. 10A****).** Second, immunofluorescence (IF) staining further demonstrated an increase of the ratio CD8⁺/Foxp3⁺CD4⁺ in the castalagin/IsoPD-1 group relative to Water/IsoPD-1 **(****FIG. 10B-C).** In E0771 cells, castalagin was associated with an increase in frequency of memory CD8⁺ T cells (CD44^{High}CD62L⁻CD8⁺ T cells) in both tumor microenvironment as well as splenocytes when comparing αPD-1 with or without CC **(****FIGs. 10D** and **10E****).**

As previously performed using avatar mice, the therapeutic effects of castalagin we tested post-FMT using NR NSCLC patients in ATB and GF conditions. Addition of castalagin alone was able to restore antitumor activity with an additive effect when combined with αPD-1 **(****FIGs. 11A-B).**

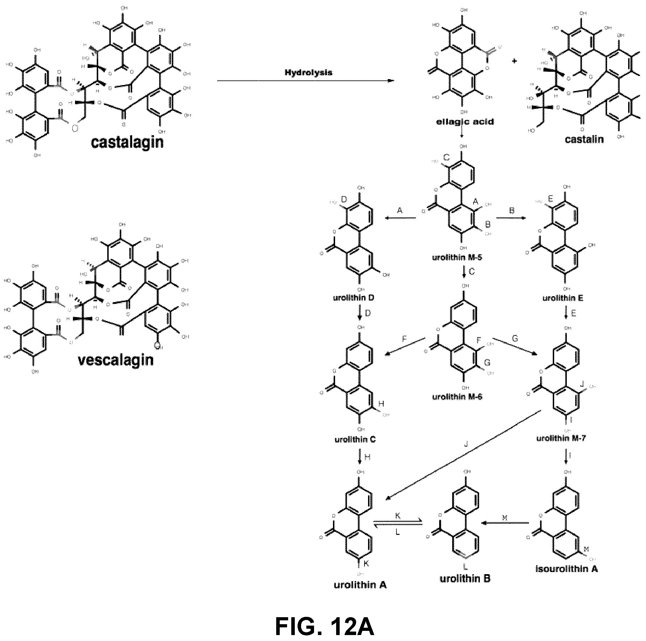

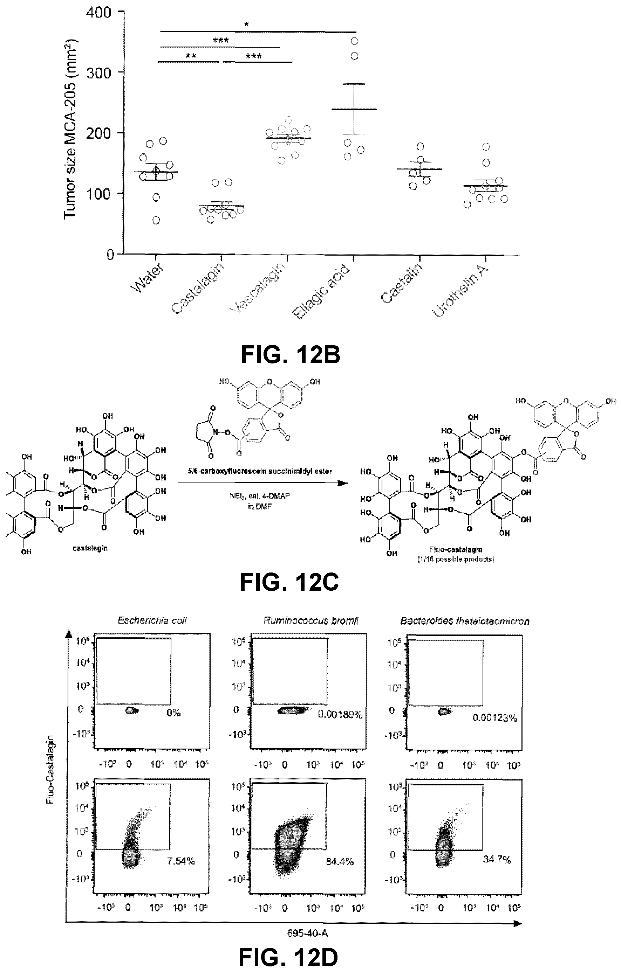

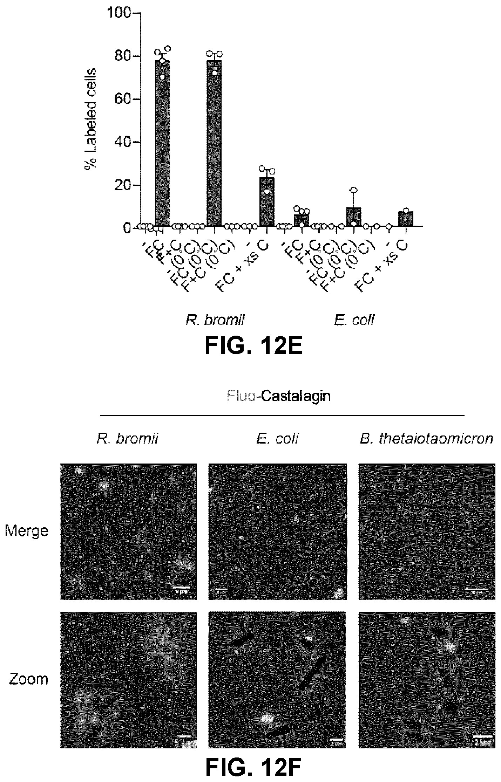

To elucidate the mechanism through which castalagin altered the gut microbiome composition and modified the microbiome, the metabolites of castalagin and its isomer vescalagin were analysed. Castalagin is hydrolyzed to an ellagic acid and castalin, ellagic acid is then further converted into urolithins by the gut microbiome **(****FIGs. 12A-12B****).** Therefore, the potential antitumor effects of some of these metabolites (ellagic acid and urolithin A) as well as that of vescalagin were individually tested. In contrast to castalagin, no antitumor effects were observed with the downstream metabolites or the isomer **(****FIG. 12B****).**

Using fluorescein-bound castalagin **(****FIG. 12C****),** it was next assessed whether castalagin is able to interact with *Ruminococcaceae.* The results depicted in **FIGs. 12D** and **12E** show that co-culturing of fluorescein-labelled castalagin with *Ruminococcus bromii* leads to labelling of a large proportion (~80%) of the bacteria, whereas less 10% and 34% of *Escherichia Coli* and *bacteroides thetaiotaomicron,* respectively, become labelled following co-culture under the same conditions. Incubation in the presence of excess (100x) unlabeled castalagin reduces the proportion of fluorescein-labelled *Ruminococcus Bromii* **(****FIG. 12E****).**

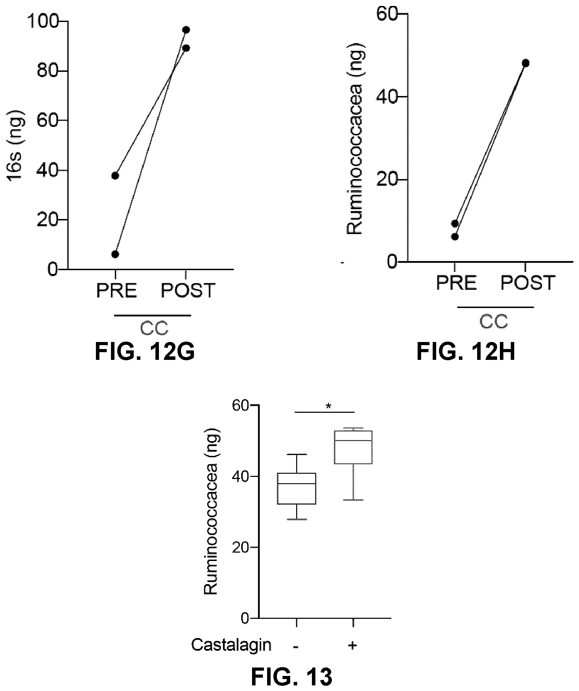

**FIGs. 12G and 12H** show that daily administration of 1.5 mg of Camu-camu for 3 weeks in two non-cancer human patients leads to an increase of the diversity (16s) and in the representation of *Ruminococcaceae* in fecal samples, consistent with the results obtained in mice.

**FIG 13** shows the results of a duplicated qRT-PCR experiment with *Ruminococcaceae-*specific primers that was performed on the feces in castalagin experiment, confirming an increase of *Ruminococcaceae* in castalagin-treated groups **(****FIG. 13****).**

## Claims

1. Castalagin for use in enhancing the anti-tumor immune response in a subject suffering from cancer.

2. The castalagin for use of claim 1, wherein the castalagin is for use in combination with an immune checkpoint inhibitor.

3. The castalagin for use of claim 2, wherein the immune checkpoint inhibitor is a Programmed cell death-1 (PD-1) inhibitor, a cytotoxic T-lymphocyte-associated antigen 4 (CTLA-4) inhibitor, or a Programmed death-ligand 1 (PD-L1) inhibitor.

4. The castalagin for use of claim 2 or 3, wherein the immune checkpoint inhibitor is a blocking antibody.

5. The castalagin for use of any one of claims 2 to 4, wherein the immune checkpoint inhibitor is a PD-1 inhibitor.

6. The castalagin for use according to any one of claims 2 to 5, wherein the subject suffers from a cancer resistant to immunotherapy.

7. The castalagin for use of any one of claims 1 to 6, wherein the castalagin is present in a plant or fruit extract.

8. The castalagin for use of claim 7, wherein the extract is a *Myrciaria dubia* extract.

9. The castalagin for use of any one of claims 1 to 6, wherein the castalagin is present in a pharmaceutical composition.

10. The castalagin for use of any one of claims 7 to 9, wherein the extract or pharmaceutical composition is formulated for delivery of the castalagin into the intestines.

11. The castalagin for use of claim 10, wherein the extract or pharmaceutical composition is formulated as a capsule.

12. The castalagin for use of any one of claims 1 to 11, wherein the cancer is melanoma, lung cancer, renal cancer, Hodgkin lymphoma, head and neck cancer, colon cancer, liver cancer, stomach cancer, myeloma, or breast cancer.

13. The castalagin for use of claim 12, wherein the cancer is lung cancer or breast cancer.

14. The castalagin for use of claim 13, wherein the lung cancer is non-small cell lung cancer (NSCLC).

15. The castalagin for use of claim 13, wherein the breast cancer is triple-negative breast cancer (TNBC).

## Patentansprüche

1. Castalagin zur Verwendung bei der Verstärkung der Antitumor-Immunantwort bei einem Patienten, der an Krebs leidet.

2. Castalagin zur Verwendung nach Anspruch 1, wobei das Castalagin zur Verwendung in Kombination mit einem Immuncheckpoint-Inhibitor bestimmt ist.

3. Castalagin zur Verwendung nach Anspruch 2, wobei der Immuncheckpoint-Inhibitor ein PD-1-Inhibitor (Programmed Cell Death-1), ein CTLA-4-Inhibitor (Cytotoxic T-Lymphocyte-Associated Antigen 4) oder ein PD-L1-Inhibitor (Programmed Death-Ligand 1) ist.

4. Castalagin zur Verwendung nach Anspruch 2 oder 3, wobei der Immuncheckpoint-Inhibitor ein blockierender Antikörper ist.

5. Castalagin zur Verwendung nach einem der Ansprüche 2 bis 4, wobei der Immuncheckpoint-Inhibitor ein PD-1-Inhibitor ist.

6. Castalagin zur Verwendung nach einem der Ansprüche 2 bis 5, wobei der Patient an einem Krebs leidet, der resistent gegen eine Immuntherapie ist.

7. Castalagin zur Verwendung nach einem der Ansprüche 1 bis 6, wobei das Castalagin in einem Pflanzen- oder Fruchtextrakt vorhanden ist.

8. Castalagin zur Verwendung nach Anspruch 7, wobei der Extrakt ein *Myrciaria-dubia*-Extrakt ist.

9. Castalagin zur Verwendung nach einem der Ansprüche 1 bis 6, wobei das Castalagin in einer pharmazeutischen Zusammensetzung vorhanden ist.

10. Castalagin zur Verwendung nach einem der Ansprüche 7 bis 9, wobei der Extrakt oder die pharmazeutische Zusammensetzung zur Abgabe des Castalagins in die Därme formuliert ist.

11. Castalagin zur Verwendung nach Anspruch 10, wobei der Extrakt oder die pharmazeutische Zusammensetzung als eine Kapsel formuliert ist.

12. Castalagin zur Verwendung nach einem der Ansprüche 1 bis 11, wobei der Krebs ein Melanom, Lungenkrebs, Nierenkrebs, Hodgkin-Lymphom, Kopf-Hals-Krebs, Darmkrebs, Leberkrebs, Magenkrebs, Myelom oder Brustkrebs ist.

13. Castalagin zur Verwendung nach Anspruch 12, wobei der Krebs Lungenkrebs oder Brustkrebs ist.

14. Castalagin zur Verwendung nach Anspruch 13, wobei der Lungenkrebs ein nicht-kleinzelliger Lungenkrebs (NSCLC) ist.

15. Castalagin zur Verwendung nach Anspruch 13, wobei der Brustkrebs ein triple-negativer Brustkrebs (TNBC) ist.

## Revendications

1. Castalagine pour l'utilisation dans l'amélioration de la réponse immunitaire antitumorale chez un sujet souffrant de cancer.

2. Castalagine pour l'utilisation selon la revendication 1, dans laquelle la castalagine est destinée à être utilisée en combinaison avec un inhibiteur de point de contrôle immunitaire.

3. Castalagine pour l'utilisation selon la revendication 2, dans laquelle l'inhibiteur de point de contrôle immunitaire est un inhibiteur de la protéine 1 de la mort cellulaire programmée (PD-1), un inhibiteur d'antigène 4 cytotoxique du lymphocyte T cytotoxique (CTLA-4), ou un inhibiteur du ligand de la protéine 1 de la mort cellulaire programmée (PD-L1).

4. Castalagine pour l'utilisation selon la revendication 2 ou 3, dans laquelle l'inhibiteur de point de contrôle immunitaire est un anticorps bloquant.

5. Castalagine pour l'utilisation selon l'une quelconque des revendications 2 à 4, dans laquelle l'inhibiteur de point de contrôle immunitaire est un inhibiteur de PD-1.

6. Castalagine pour l'utilisation selon l'une quelconque des revendications 2 à 5, dans laquelle le sujet souffre d'un cancer résistant à l'immunothérapie.

7. Castalagine pour l'utilisation selon l'une quelconque des revendications 1 à 6, dans laquelle la castalagine est présente dans un extrait de plante ou de fruit.

8. Castalagine pour l'utilisation selon la revendication 7, dans laquelle l'extrait est un extrait de *Myrciaria dubia*.

9. Castalagine pour l'utilisation selon l'une quelconque des revendications 1 à 6, dans laquelle la castalagine est présente dans une composition pharmaceutique.

10. Castalagine pour l'utilisation selon l'une quelconque des revendications 7 à 9, dans laquelle l'extrait ou la composition pharmaceutique est formulé(e) pour l'administration de la castalagine dans les intestins.

11. Castalagine pour l'utilisation selon la revendication 10, dans laquelle l'extrait ou la composition pharmaceutique est formulé(e) sous forme de capsule.

12. Castalagine pour l'utilisation selon l'une quelconque des revendications 1 à 11, dans laquelle le cancer est un mélanome, un cancer du poumon, un cancer des reins, un lymphome hodgkinien, un cancer de la tête et du cou, un cancer du côlon, un cancer du foie, un cancer de l'estomac, un myélome, ou un cancer du sein.

13. Castalagine pour l'utilisation selon la revendication 12, dans laquelle le cancer est un cancer du poumon ou un cancer du sein.

14. Castalagine pour l'utilisation selon la revendication 13, dans laquelle le cancer du poumon est le cancer du poumon non à petites cellules (CPNPC).

15. Castalagine pour l'utilisation selon la revendication 13, dans laquelle le cancer du sein est le cancer du sein triple négatif (CSTN).
